(19) Europäisches Patentamt
European Patent Office
Office européen des brevets

(11) EP 1 149 836 B1

(12) EUROPEAN PATENT SPECIFICATION

(45) Date of publication and mention of the grant of the patent:
**26.02.2003 Bulletin 2003/09**

(51) Int Cl.[7]: **C07D 489/08**, A61K 31/485, A61P 23/00

(21) Application number: **00303532.6**

(22) Date of filing: **27.04.2000**

# (54) Polynalbuphine derivatives

Polynalbuphinderivat

Dérivé de polynalbuphine

(84) Designated Contracting States:
**AT BE CH CY DE DK ES FI FR GB GR IE IT LI LU MC NL PT SE**
Designated Extension States:
**AL LT LV MK RO SI**

(43) Date of publication of application:
**31.10.2001 Bulletin 2001/44**

(73) Proprietor: **Hu, Oliver Yoa-Pu**
**Taipei City (TW)**

(72) Inventors:
- **Hu, Oliver Yoa-pu**
  **Tapei City (TW)**
- **Hsiong, Cheng-Huei**
  **Tapei City (TW)**

(74) Representative: **Crisp, David Norman et al**
**D. YOUNG & CO.**
**21 New Fetter Lane**
**London EC4A 1DA (GB)**

(56) References cited:
**EP-A- 0 170 090**
**EP-A- 0 615 756**
**FR-A- 2 184 065**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 1 149 836 B1

## Description

## BACKGROUND OF THE INVENTION

1. Field of the invention

This invention relates to a long acting analgesic polynalbuphine derivative, a process for making the same, and a pharmaceutical composition containing polynalbuphine derivative.

Description of the related art

New types of opiods drugs, such as Buprenorphine, Nalbuphine, Butorphanol, so-called narcotic agonist-antagonist analgesics, have been developed. These drugs exhibit a dual action of agonist and antagonist on opiods-receptors as reported by Schmidt, W.K. et al ( Drug Alcohol Depend. 14, 339, 1985), where it was pointed out that these dual action drugs not only had a high affinity to opium receptors but also served as an anatagonist. For example, Nalbuphine is an antagonist for a Mu receptor and an agonist for a Kappa receptor. These agonist/ antagonist drugs have improvement on untoward effects of opiods drugs, such as addiction and respiratory suppression.

Shafer, S.L. et al. have investigated the analgesic potency of narcotic agonist-antagonist analgesics. They have found that the dose needed to elicit the same analgesic effect for Morphine and Nalbuphine are 10 mg; for Buprenorphine is 0.3 mg; and for Butorphanol is 2 mg (Anesthesiology, 74, 53, **1991**). According to the publication by Schmidt, W.K. et al, supra(1985) , Nalbuphine is the most widely used thereamong and has excellent therapeutic efficacy. After continuous use of Nalbuphine for 6 months, no significant addiction and synergistic effect was found, but only slight respiratory inhibition. In clinical use, Nalbuphine is safer than traditional narcotic analgesics.

An ideal analgesic should exhibit short onset time, long acting, potency, and no adverse effects, such as addiction, inhibition of the cardiac or cardiovascular system, and respiratory inhibition, which were suggested by Bovill, J.G. et al.(Drugs, 33, 520, **1987**). For the relief of pain, local anaesthetics like Xylocaine or Bupivacaine can only be applied to restricted areas. In addition, local anaesthetics are short acting and even when they are given intracerebroventricularly, the duration of action hardly exceeds 6 hours. Therefore, for severe and acute pain caused by cardiac, pulmonary, abdominal, osteopathia, and obstetrical surgery, severe burn injury and terminal stage of cancer, local anaesthetics are not satisfactory.

Non-narcotic analgesics, such as Acetaminophen and aspirin, can relieve pain of only low intensity, such as pain due to headache or toothache, but they do not help in the case of serious pain. Bovill, J.G. et al. reported in Drugs volume 33, page 520 that a strong narcotic analgesic drug should have action on Mu receptor in central nervous system. Hayes, A.G. et al.(Br. J. Pharmacol. Vol 79, 731, **1983**) have reported that all narcotic analgesics exhibit the same disadvantages with respect to addiction, synergistic effect, and respiratory inhibition. In addition, the duration of action of the narcotic analgesics is somewhat short. Normally, to maintain the analgesic effect, the dosing interval needs to be set at 3-5 hours. Even when the agent is administered to the spinal marrow, the duration of action could not be longer than 48 hours.

According to Schmidt, W.K. et al., supra(1985), and clinical cases, it is found that Nalbuphine was widely used in those narcotic agonist-antagonist analgesics. Nalbuphine has been found to be effective in controlling severe and deep pain caused by cardiac, pulmonary, abdominal, osteopathia, and obstetrical surgery, severe burn injury and the terminal stages of cancer via various parenteral administration routes, such as intramuscular, intravenous, intrathecal and intracerebroventricular. However, the duration of analgesic action thereof was short. Wang, J. J. et al. revealed data in 1985 Ma Tsui Hsueh Tsa Chi, volume 23, page 3, regarding the duration for different administration routes for Nalbuphine. For intravenous parenteral administration, the analgesic effect was maintained at 3 to 5 hours. For intracerebroventricular, the analgesic effect was maintained at 6 to 8 hours.

Nalbuphine analogue compound was found in US patent No. 4,673,679, which discloses 3-morphine derivatives, in which the substitution group R was methyl, propyl, methyl propyl, methyl cyclopropyl,or cyclopentyl; R1 and R2 were hydrogen and carboxyl groups, respectively, R2 could also be lactone or methylene group; R3 and R4 were hydrogens or bonded to oxygen; and R5 was an alkanoyl group containing 2 to 18 carbons, benzoyl, or halides. It also discloses pharmaceutical dosage provided for sublingual, buccal, or nasal administration.

WO patent No. 82/03,768 revealed that narcotic antagonists, narcotic analgesics and related compounds, such as Nalbuphine, were prepared with a pH adjusting agent, jelling agent, emulsifier, emollient, cellulose derivatives, and a Tween 80 formed agent, such as nasal solution, nasal suspension, nasal ointment, and nasal jelly. European patent A1 No. 170,090 revealed a substituted benzolate ester prodrug derivative of 3-hydroxymorphinan. The compound is useful as analgesics or narcotic antagonists, and provides dosage forms for capsules, tablets, suspensions, suppositories, and injections. US patent No. 4,722,928 revealed N-oxide prodrug derivative of 3-hydroxymorphine and partial morphinan analgesics, agonist-antagonists, and narcotic antagonists which are useful for therapeutic effects.

Those compounds were prepared in various dosage forms with diluents, carriers such as magnesium stearate, cellulose derivatives, lactose, starch for tablets, powder, capsules, suspensions, syrups etc. for orally administration dosage forms. In view of the foregoing, it is apparent that all of these patents contained Nalbuphine but did not include polynalbuphine derivatives, and nothing disclosed in these patents suggests a process for making the polynalbuphine derivatives.

It has been reported in JACS volume 97, page 3515 (1975) that the use of Mg(OCOCF3 )2 and alkaline reagents, or the use of halides in acids to react with Nalbuphine can lead to formation of Nalbuphine containing esters. In **1981**, Synth. Comm. volume 11, page 121 reported the use of 1-flouro-2,4,6-trinitrobenzene and N,N-dimethylaminopyridine to synthesize Nalbuphine containing ester. In **1983**, Bull. Chem. Soc. Jpn. volume 56, page 639, aromatic Nalbuphine containing ester was prepared by mixing pyridine, 1-chloro-2,4-nitropyridine and an aromatic fatty acid. In **1984**, Tetraheder Lett. volume 25, page 4943, reactants such as N,N-dimethylaminopyridine, and di-2-pyridyl carbonate were used. However, these methods only described the product of mono-Nalbuphine containing ester.

European patent application EP-0615 756-A1 discloses long acting analgesic Nalbuphine prodrugs including a process for the preparation, and phamaceutical composition comprising nalbuphine prodrugs. These prodrugs are long-acting injectable analgesics when given intramuscularly, subcutaneously, intracerebroventricularly or in the mammal via the oral, sublingual, buccally or percutaneous route or other administration routes.

The nalbuphine prodrugs were synthesized by esterification of nalbuphine base with acid chlorides or acid anhydrides of saturated fatty acids or unsaturated fatty acids with various lengths.

## SUMMARY OF THE INVENTION

Therefore, it is an object of the present invention to provide a polynalbuphine derivative that possesses long acting analgestic effect in relieving pain.

It is another object of the present to provide a process for making the polynalbuphine derivative.

It is yet another object to provide a pharmaceutical composition that contains the polynalbuphine derivative and that possesses the aforementioned properties, such as long acting, short onset time, no addiction, no inhibition of cardiac or cardiovascualr system, no respiratory inhibition, etc..

According to one aspect of the present invention, a polynalbuphine derivative contains a compound having the following formula (I):

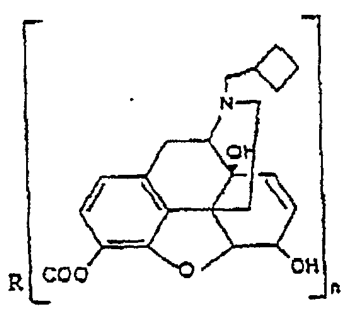

(I)

wherein n is an integer number ≧ 2; and R is a substituted or unsubstituted hydrocarbyl group having 1 to 40 carbon atoms.

According to another aspect of the present invention, a process for producing a compound of formula (I) comprises the step of reacting Nalbuphine or Nalbuphine salt with a carbonyl group containing compound which is selected from a group consisting of a polybasic carboxylic acid, a polybasic carboxylic acid anhydride, and a polybasic carboxylic acid derivative containing at least two carbonyl chloride groups.

According to yet another aspect of the present invention, a pharmaceutical composition comprises a compound of formula (I) and a pharmaceutically acceptable carrier.

## BRIEF DESCRIPTION OF THE DRAWINGS

In drawings and tables which illustrate embodiments of the invention,

Table 1 depicts the physical properties of polynalbuphine derivatives of formula (I) embodying this invention;

Table 2 depicts the half-lives of a soft drug containing the polynalbuphine derivative of formula (I) in various animal blood tests for rat and rabbit;
Table 3 depicts the half-lives of the soft drug in various blood tests for dog and human;
Table 4 depicts the excipients in a pharmaceutical composition containing the polynalbuphine derivative of formula (I);
Table 5 depicts the stability test for the pharmaceutical compositions;
Table 6 depicts pharmacokinetic parameters of dinalbuphine obtained upon injection of Sebacoyl dinalbuphine (SDN ) into five Beagle dogs;
Table 7 depicts pharmacokinetic parameters of dinalbuphine obtained upon injection of different doses of Sebacoyl dinalbuphine (SDN ) into the same Beagle dogs;
Table 8 illustrates a number of MonoNalbuphine esters;
Table 9 illustrates a number of polynalbuphine esters;
Figure 1 depicts the metabolism of the polynalbuphine derivative of formula (I);
Figure 2 depicts in vitro release of Nalbuphine hydrochloride from vehicles (average ± standard deviation, N = 6);
Figure 3 depicts in vitro release of Nalbuphine freebase from vehicles (average ± standard deviation, N = 6);
Figure 4 depicts the analgesic effect of Nalbuphine HCl, Morphine HCl and Buprenorphine HCl;
Figure 5 depicts the analgesic effect after intramuscular injection of several nalbuphine monoesters to rats (N = 6);
Figure 6 depicts the analgesic effect after intramuscular injection of Nalbuphine HCl to rats;
Figure 7 depicts the analgesic effect after intramuscular injection of Nalbuphine HCl to Guinea pigs;
Figure 8 depicts the analgesic effect after intramuscular injection of sebacoyl dinalbuphine (SDN) to rats;
Figure 9 depicts comparison of the duration of Nalbuphines after intramuscular injection to rats for sebacoyl dinalbuphine (SDN) and nalbuphine salt; and
Figures 10 and 11 depict comparison of the plasma concentrations of Nalbuphines after intramuscular injection to dogs for sebacoyl dinalbuphine (SDN) and nalbuphine salt.

## DETAILED DESCRIPTION OF THE PREFERRED EMBODIMENTS

The polynalbuphine derivative of this invention contains a compound having the following formula (I):

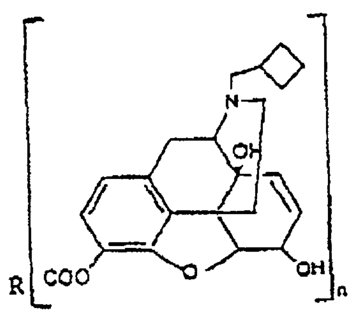

(I)

wherein n is an integer number ≧ 2; and R is a substituted or unsubstituted hydrocarbyl group having 1 to 40 carbon atoms.

The hydrocarbyl group is preferably an aliphatic group consisting of linear or branched alkyl or alkylene having 1 to 40 carbon atoms, a substituted or unsubstituted alicyclic group having 3 to 12 carbons atoms, or a substituted or unsubstituted aromatic group having 6 to 40 carbon atoms, and n represents an integer in the range of 2 to 4. The hydrocarbyl group is more preferably a linear or branched alkyl or alkylene group having 20 to 35 carbon atoms, an alicyclic group having 3 to 8 carbon atoms and unsubstitutued or sustituted with a linear or branched alkyl or alkylene having 1 to 8 carbon atoms, or a phenyl unsubstitutued or substituted with an linear or branched alkyl or alkylene having 1 to 8 carbon atoms

The polynalbuphine derivative of formula (I) is prepared by a process including the step of reacting Nalbuphine or Nalbuphine salt, such as Nalbuphine hydrochloride, with a carbonyl group containing compound which is selected from a group consisting of a polybasic carboxylic acid, a polybasic carboxylic acid anhydride, and a polybasic carboxylic acid derivative containing at least two carbonyl chloride groups.

The polybasic carboxylic acid is preferably selected from a group consisting of adipic acid, suberic acid, sebacic acid, dodecanedic acid, docosanic acid, 3,3-dimethyl glutaric acid, 1,3-cyclohexane dicarboxylic acid, isophthalic acid, phthalic acid, trimesic acid, 1,3,5-cyclohexane tricarboxylic acid, pyromellitic acid, and the like.

The polybasic carboxylic acid anhydride is preferably selected from a group consisting of adipic acid anhydride, suberic acid anhydride, sebacic acid anhydride, dodecanedic acid anhydride, docosanic acid anhydride, 3,3-dimethyl glutaric acid anhydride, 1,3-cyclohexane dicarboxylic acid anhydride, isophthalic acid anhydride, phthalic acid anhydride, trimesic acid anhydride, 1,3,5-cyclohexane tricarboxylic acid anhydride, pyromellitic acid anhydride, and the like.

The polybasic carboxylic acid derivative is selected from a group consisting of adipoyl chloride, suberoyl chloride, sebacoyl chloride, dodecanedoyl chloride, docosanioyl chloride, 3,3-dimethyl glutaric diacid chloride, 1,3-cyclohexane dicarboxylic acid chloride, isophthaloyl chloride, phthaloyl chloride, trimesoyl chloride, 1,3,5-cyclohexane tricarboxylic acid chloride, pyromellitoyl chloride, and the like.

The reaction of Nalbuphine or Nalbuphine salt with the carbonyl group containing compound in the aforesaid process is preferably carried out in the presence of an organic solvent which is selected from a group consisting of methylene chloride, ethylene chloride, and the like, and which is preferably methylene chloride.

Nalbuphine hydrochloride is most preferably employed as a reagent in the aforesaid process among the Nalbuphine salts. A neutral agent is added in the reaction mixture for neutralizing hydrochloride disassociated from the Nalbuphine hydrochloride, and for controlling the pH value of the reaction mixture as well during the reaction. The neutral agent is selected from a group consisting of a dimethylamine, trimethylamine, 2-pyridine carbonate, N,N-dimethylaminopyridine, N,N'-dicyclo-hexylcarbodiimide, 4-dimethylaminopyridine, pyridine, HOBT, and the like, and is preferably trimethylamine. The pH value of the reaction mixture is preferably controlled in a range of between 8 to 10. Reaction temperature also affects production yield significantly, and is preferably controlled to be between -10°C to 100°C. When aromatic fatty acids are employed, the reaction temperature is preferably controlled to be between 0°C to 80°C, and is more preferably less than room temperature and above -10°C. When aromatic fatty acid halides are employed, the reaction temperature is preferably controlled to be between -10°C to 50°C, and is more preferably less than room temperature and above -10°C.

The number of carboxylic groups contained in the carbonyl group containing compound is required to be equal or greater than the n value of the desired polynalbuphine derivative of formula (I).

The mole ratio of the nalbuphine or nalbuphine salt to the aforesaid carbonyl group containing compound is preferably greater than 1:1 when n is equal to 2, or greater than 2:1 when n is equal to 3, or greater than 3:1 when n is equal to 4. In the case of producing the polynalbuphine derivative of formula (I) with n being equal to 2, when the mole ratio of the nalbuphine to the carbonyl group containing compound is 2:1, the yield is 80%; when the mole ratio of the nalbuphine to the carbonyl group containing compound is 2.2:1, the yield is 85%; and when the mole ratio of the nalbuphine to the carbonyl group containing compound is 2:1.15, the yield can reach up to 90%.

The polynalbuphine derivative of formula (I) can also be prepared by another process which includes reacting a polynalbuphine derivative of formula (I), which has a lower number of n value than that of the desired product and which contains at least a carboxylic group, with the nalbuphine or nalbuphine salt. For instance, in the case of preparing the polynalbuphine derivative of formula (I) with n value being equal to 3, the polynalbuphine derivative of formula (I) with n value being equal to 2 and with at least a carboxylic group can be employed in the reaction mixture to react with the nalbuphine or nalbuphine salt to form the desired product.

To prevent decomposition by solvent, such as water and methanol, or to avoid using environmentally unfavorable solvents, such as THF, hexane, ether, and the like, or to avoid using solvent such as methylene chloride and ethylene chloride which exhibits slow production rate in the course of crystallization, multiple solvents and multiple stages are employed in the purification step in the process of the present invention. For instance, ethanol solvent *is* used in a first stage, and ethanol/ propanol multiple solvent is used in a second stage in a two-staged purification step in the process of the present invention.

Table 1 shows the physical characteristics of some of the polynalbuphine derivatives of formula (I), such as Adipoyl dinalbuphine ester, suberoyl dinalbuphine ester, sebacoyl dinalbuphine ester, Dodecanedioyl dinalbuphine ester, Isophthaloyl dinalbuphine ester, Phthaloyl dinalbuphine ester, 1,3-cyclohexane diacid dinalbuphine ester, Docosanodic dinalbuphine ester, 3,3-Dimethylglutarioyl dinalbuphine ester, Trimesoyl trinalbuphine ester, 1,3,5,-Cyclohexane triacid trinalbuphine ester, Pyromellitoyl tetranalbuphine ester, which were analyzed by NMR, IR, UV, GC-MS. Those compounds were prepared to form various dosage forms for oral, topical route or parenteral administration, such as intramuscular, intracerebroventricularly intravenous, and intrathecal.

**DESIGN OF POLYNALBUPHINE DERIVATIVE SOFT DRUG**

The polynalbuphine derivative of formula (I) can be formulated into a "soft drug". The item, "soft drug", is different from a prodrug or a hard drug. Generally, hard drugs are the products that were prepared by the synthesis from starting materials, or purified from a natural product. Those hard drugs usually have some activity, and are combined with mineral toxicity. The prodrugs were developed via studies of pharmacokinetic and pharmacodynamic. These prodrugs were metabolized into two parts, namely an activity part, and unknown part. For a Nalbuphine soft drug, it

was necessary to know the active form of the drug (Nalbuphine) first, then a nontoxic-prodrug was synthesized from an active form of Nalbuphine with a non-active and nontoxic compound. As shown in Figure 1, the polynalbuphine derivatives of formula (I) of Nalbuphine soft drugs are metabolized to release bioactivity Nalbuphine in the animal body. The polynalbuphine derivative can be converted into Nalbuphine and non-active and nontoxic metabolites by metabolism. Therefore, the Nalbuphine soft drug is different from common Nalbuphine prodrugs or hard drugs. Since it was not converted into toxic metabolites, the soft drug design was the safest way.

## PROPERTIES OF POLYNALBUPHINE DERIVATIVE

I. For rat experiments

(a) Dinalbuphine sebacoyl ester(SDN) was dissolved in acetonitrile, the concentration was 7.75 mg/ml.
(b) 250 ml of whole blood was collected from rat administered with the thus formed dinalbuphine sebacoyl ester soft drug, and was added with anticoagulant.

II. For rabbit experiments

(a) Dinalbuphine ester sebacoyl ester was dissolved in acetonitrile, the concentration was 5.2 mg/ml.
(b) 150 ml of whole blood was collected from rabbit administered with the thus formed dinalbuphine sebacoyl ester soft drug, and was added with anticoagulant.

III. For dog experiments

(a) Dinalbuphine sebacoyl ester was dissolved in acetonitrile, the concentration was 12.5 mg/ml.
(b)200 ml of whole blood was collected from dog administered with the thus formed dinalbuphine sebacoyl ester soft drug, and was added with anticoagulant.

IV. for human experiments

(a) Dinalbuphine sebacoyl ester was dissolved in acetonitrile, the concentration was 10 mg/ml.
(b) 250 ml of whole blood was collected from human administered with the thus formed dinalbuphine sebacoyl ester soft drug, and was added with anticoagulant.

All blood collections were analyzed by HPLC. Half-lives in various animals tests and the human test are shown in Table 2 and Table 3. For rat, the half life was 2.8 minutes, rabbit was 7.0 minutes, dog was 27.8 minutes, and human was 9.0 minutes. The converted time, which converts 90% dinalbuphine sebacoyl ester(SDN) into Nalbuphine, for rat was 10 minutes, for rabbit was 25 minutes, for dog was 95 minutes, and for human was 30 minutes.

## LONG ACTING PHARMACEUTICAL COMPOSITION

Gelders, Y.G. reported in Int. Clin. Psychophacol. volume 1, page 1 (**1986**), regarding Haloperidol decanoate as long acting prodrug for Haloperidol. Intramuscular parenteral administration of the prodrug was given, and the analgesic effect was extended from 2 to 4 times a day, to 1 to 2 times a month. In **1987**, Norman T.R. reported in Int. Clin. Psychopharmacol. Volume 2, pages 299-305, regarding the preparation of Fluphenazin decanoate from Fluphenazin. In **1988**, Hinko, C.N. reported in Neuropharmacology volume 27, pages 475 to 483, regarding the preparation of ester of Nipectic acid. In **1988**, Broekkamp C.L. reported in J. Pharm. Pharmacol. volume 40, pages 434 to 437, regarding the preparation of Nicotinoyl morphine ester from Morphine. Joshi, J.v. et al. reported in **1989**, Steroids, volume 53, pages 751 to 761, regarding a precursor preparation of Northisterone enanthate, where the dosing can be set longer to two months.
In general, to maintain the long acting therapeutic efficacy, various dosage forms were prepared by which

drugs may be esterified, and are suspended in an oil vehicle to form parenteral suspension administration. When it is administered in animal body, the release rate of those drugs may slow down, which is caused by factors such as increased solubility in fat or lesser blood flow. In these cases, the dosing interval can be set longer by virtue of the long acting effect. In **1986**, Gelders reported in Int. Clin. Psychophacol, volume 1, page 1, and Hinko, C.N. et al. reported in Neuropharmacology volume 27, page 475, in 1998, the addition of sesame oil or soybean oil to precursor Haloperidol decanoate to form a controlled dosage form. However, simply suspending the drug in the oil sometimes could achieve quick release. For instance, Tanaka, T. reported in **1974** in Chem. Pharm. Bull. volume 22, pages 1275 to 1284, that for intramuscular administration of a Testosterone suspension, the release of Testosterone was found to be quick. In **1990**, Titulaer, H.A.C. reported in J. Phar m. Pharmacol. volume 42, pages 810 to 813, that suspending artemisinin in parenteral oil makes various dosage forms for intramuscular, intravenous, oral, and rectal administration. The drug was released promptly, In **1994**, Zuidema, Z. et al. reported in International J. of Pharmaceutics, volume 105, pages 189 to 207, that the rate and extent of dosage forms for parenteral administration are very erratic and variable. In general, any attempt to suspend drug in an oil vehicle for the purpose of obtaining long acting dosage forms required the consideration of physical solubility, stability, and release rate from such vehicle.

According to the aforementioned studies, a pharmaceutical composition which contains a suspended or dissolved esterified dosage form in oil vehicle, did not certainly achieve the long duration therapeutic effect. It required further testimony and study. These prior art disclosed some long acting pharmaceutical composition comprising active ingredients for hormones or antipsychotic drugs. Those chemical structures of the active ingredients mentioned in the prior art, are different from polynalbuphine derivatives which serve as narcotic agonist-antagonists. Therefore, the prior art do not have any teaching about the present invention. The present invention took consideration of these factors, and many obstacles were overcome to achieve the goal of extending the duration of action.

The present invention also provides long acting dosage form for polynalbuphine derivative. It employed a method which adds polynalbuphine derivative to injectable oil vehicle, or to phosphate buffer, with the addition of common excipients to form a controlled release dosage form. In the present invention, the injectable oil vehicles were selected from sesame oil, ethylester of peanut oil, or soybean oil. The common excipients were selected from methyl paraben, propyl paraben, BHA, BHT, cremophore EL, pluronic, solutol, or Span.

A few problems about the processing of long acting dosage forms needed to be overcome. One of the problems concerns dispersing of polynalbuphine derivative in oil vehicle could cause precipitation, crystallization turbidity. Another problem was that the total amount of active ingredient dissolved was low. Even when an excipient was added, the total amount of polynalbuphine derivative dissolved was low, such as between 8.5% and 16.8%, and was still far away from a recognized ideal concentration 95%. Therefore, the strategy of using multiple excipients, or raising temperature was adopted in the process. The result was not satisfactory. Although the total amount of polynalbuphine derivative was increased, some are far away from 95%, others over 95% but unfit for parenteral administration.

As shown in Table 4, favorable excipients that form long acting dosage forms of polynalbuphine derivative are listed. One or more than one excipients were selected from methyl paraben, propyl paraben, benzyl alcohol and span. Those excipients can solve the problems between polynalbuphine derivative and the oil vehicle, such as precipitation, crystallization turbidity. Table 5 shows that the amounts of excipients added were between 0.01 to 46%, and the total amount of polynalbuphine derivative dissolved was more than 95%.

The pharmaceutical compositions comprising polynalbuphine derivative suspended or dissolved in respective oils and phosphate buffer were tested in animals such dogs, and Sprague Dawley rats for analgesic effect by intramuscular, intravenous parenteral administration.

## Selection Of Injectable Oil Vehicle For Controlled Release Dosage Form

(1) materials

Sesame oil(Sigma, MO, USA), soybean oil(Sigma, MO, USA), and peanut oil(Chun Sin, Taipei, Taiwan) were tested in the present invention as the oil vehicle for polynalbuphine derivative. Phosphate buffer was prepared by adding 1.9 g of monobasic potassium phosphate, 8.1 g of dibasic sodium phosphate, and 4.1 g of sodium chloride to 1 liter of water to make an isotonic buffer solution of pH 7.4.

(2) Experiment

Each experimental group has 6 samples. Into a dialysis bag was placed 50 mg(0.127 mmole) of Nalbuphine hydrochloride, or 45 mg Nalbuphine free base(0.127 mmole). 1 ml of the previously mentioned oil vehicle or phosphate buffer was added. Then, the solution was filled into a dialysis bag. The cut off for the dialysis bag was 12,000 -14,000 molecular weight. 250 ml of an iodine flask containing 150 ml of phosphate buffer was placed into the dialysis bag. Inside the flask, a magnetic stirring bar(Fargo, Taipei, ROC) was placed. The dialysis proceeded at a stirring speed of

500 rpm, and the release rate of Nalbuphine from each preparation was measured. A UV spectrophotometer(Shimadzu, Kyoto, Japan) was used to detect the Nalbuphine content released from each preparation.

(3) result

The release profiles of Nalbuphine from each preparation (the oil vehicles and the phosphate buffer) are listed in Figs. 2 and 3. As shown in Fig. 2, the preparation with sesame oil as the vehicle has the slowest release rate($p<0.05$) and there is no significant difference within the other three preparations after a period of between 1 and 11 hours. Fig. 2 also shows that the amount of released Nalbuphine free base was less in the preparation with sesame oil than with peanut oil and the phosphate buffer after a period of between 17 and 28 hours ($p<0.05$).

Fig. 3 shows the amount of Nalbuphine released from preparation(A) Nalbuphine hydrochloride dissolved in sesame oil, preparation(B) Nalbuphine hydrochloride dissolved in phosphate buffer, and preparation (C) Nalbuphine free base dissolved in sesame oil. In the first 3 hours, the release in (B ) is greater than (A ) , and the release in (A ) is greater than(C). After a period of between 3 and 28 hours, the release in (A ) is greater than(C).

<u>**Pharmacodynamic Study For Long Acting Preparation Of MonoNalbuphine Derivative**</u>

(1) animal

Sprague Dawley rats (175 to 225 g) were used. Each group consisted of 6 rats, and each rat was injected once intramuscularly on the rear leg.

(2) materials

A. Dose response curve for analgesic effect

(a) Nalbuphine hydrochloride, doses of 100 mg/kg, 10 mg/kg, 1 mg/kg, 0.5 mg/kg , 0.1 mg/kg, 0.05 mg/kg, 0.01 mg/kg were used.
(b) Morphine hydrochloride, doses of 10 mg/kg, 5 mg/kg, 1 mg/kg, 0.5 mg/kg, 0.1 mg/kg, 0.05 mg/kg, 0.01 mg/kg were used.
(c) Buprenorphine hydrochloride, doses of 100 mg/kg, 10 mg/kg, 5 mg/kg, 1 mg/kg, 0.5 mg/kg, 0.1 mg/kg, 0.01 mg/kg were used.

B. Pharmacodynamic study for MonoNalbuphine derivative

(a) 25 micromole/2.8 ml of Nalbuphine hydrochloride in saline and 25 micromole/2.8 ml of Nalbuphine base in sesame oil were used as control groups. The dose for each rat was 25 micromole per kg, intramuscularly.
(b) 25 micromole/2.8 ml of MonoNalbuphine derivatives, such as propionyl MonoNalbuphine ester, enanthyl MonoNalbuphine ester, pivalyl MonoNalbuphine ester, benzoyl MonoNalbuphine ester, decanoyl MonoNalbuphine ester were dissolved and were used to form controlled release dosage forms. The dose for each rat was 25 micromole per kg, intramuscularly.

(3) Experiment

In the experiment, a circulating cold ethanol bath with a temperature maintained at -20°C was set up. After dosing, the rat tail(1/3 from the tip) was immersed in the bath. The latency for the rat to flick its tail from the bath was measured to be the analgesic threshold. The effect of various opium alkaloid preparations can be determined with this test.

The analgesic effect can be calculated as follows:

analgesic effect (%) = (flicking time after dosing -

flicking time before dosing ) /

(test terminated time - flicking

time before dosing ) X 100%

At 35, 25, 15 minutes before dosing, Male Sprague Dawley rat (175 -225 g) was tested to measure the basic response latency. The time to stop the experiment was set at 40 seconds to prevent the tail from cold sore. No cold sores were found within 40 sec. Five minutes after the drug was given to the rat, the flick test was performed every 10 minutes or more.

(4) result

A. Dose response curve

The maximal analgesic effect for intramuscularly injection was found at 10 mg/kg for Morphine hydrochloride, 100 mg/Kg for Nalbuphine hydrochloride, and 100 µg/kg for Buprenorphine. The dose response curve is shown in Figure 4.

B. Pharmacodynamic study

Fig. 5 shows that the analgesic duration of Nalbuphine free base is significantly shorter than that from each preparation of MonoNalbuphine derivatives.

**Pharmacodynamic Study For Long Acting Preparation Of Polynalbuphine derivative**

(1) animal

Six male Sprague Dawley rats (175 to 225 g), and six male guinea pig(200 to 250 g) were used.

(2) material

(a) Nalbuphine hydrochloride, doses for rats ware 15.625 µM/kg, 62.5 µM/kg, 250 µM/kg. Dose for guinea pigs were 500 µM/kg, 250 µM/kg.

(b) Nalbuphine hydrochloride parenteral dosage forms, Nalbuphine hydrochloride was dissolved in 0.9% normal saline.

(c) Dinalbuphine sebacoyl ester, doses were 7.8125 µM/kg, 31.25 µM/kg, 125 µM/kg.

(d) 50 mg/ml dinalbuphine sebacoyl ester parenteral dosage forms, dinalbuphine sebacoyl ester was dissolved in sesame oil.

(3) result

As shown in Figures 6 and 7, significant analgesic effects for intramuscular injection were found at 15.625, 62.5, 250 µM/kg for rats and 500 µM/kg and 250 µM/kg for guinea pigs for Nalbuphine hydrochloride. As shown in Figure 8, the significant analgesic effects were proportional to the dose administered for intramuscular injection, and were found at 7.8125 µM/kg, 31.25 µM/kg, 125 µM/kg for rats for sebacoyl dinalbuphine. The analgesic effect was extended to 4-5 days, when the dose administered was below 125 µM/kg. For clinical experience, the analgesic effect maintained 4 to 5 days for intramuscular injection was found at 20 mg/65 kg (0.308 mg/kg) dose of Nalbuphine hydrochloride that was reported by Schmidt, W.K. et al( Drug Alcohol Depend. 14, 339, 1985). The same level of analgesic effect for intramuscular injection to rats was required at 15.625 µM/kg(6.165 mg/kg).

As shown in Figure 9, a 50% analgesic effect was extended to 88 hours for intramuscular injection of 125 µM/kg of sebacoyl dinalbuphine. The analgesic effect of sebacoyl dinalbuphine was 8.8 times of Nalbuphine hydrochloride. Therefore, the analgesic effect is estimated 4-5 days when 125 µM/ kg of sebacoyl dinalbuphine ester is administered to a human body.

On clinic, the analgesic effect was extended 4 to 5 hours for a single dose of an intramuscular injection at 20 mg/65 kg(0.308 mg/kg) of Nalbuphine hydrochloride. The amount of the aforesaid injection was about 10 ml. However, the analgesic effect could not be maintained 4 to 5 days even upon administration of 88 ml of Malbuphine hydrochloride because of metabolism. Besides, such parenteral volume was not ideal for a single dose. This invention made an improvement. By diluting a single dose of sebacoyl dinalbuphine in 7.15 ml sesame oil form, parenteral administration could maintain the analgesic effect 4 to 5 days. This was owing to multiple stages of release of Nalbuphine from

Polynalbuphine derivative, and such effect was adequate enough for the treatment of acute and chronic pain.

**Pharmacokinetic Study For Long Acting Preparation of Polynalbuphine derivative In Beagle Dog**

(a) method

Six Beagle dogs were used. Five Beagle dogs were intramuscularly injected with 30 mg/kg long acting sebacoyl dinalbuphine ester oily parenteral administration respectively, and another one was injected with 100 mg/kg long acting sebacoyl dinalbuphine ester oily parenteral administration. Blood samples were collected through the veins in front limbs of the dogs at 1, 2, 6, 24, 30, 48, 54, 72, 78, 96, 102, 120, 168, 192 hours. The concentrations of Nalbuphine and sebacoyl dinalbuphine ester in plasma were determined. The relationship between time and concentration was analyzed by PCNONLIN computer program. Two-compartment model and pharmacokinetic parameters were calculated to depict absorption, distribution, metabolism, and excretion of drugs in the dogs.

(b) result

As shown in Figures 10 and Figure 11, the absorption of sebacoyl dinalbuphine ester in injection oil vehicle was presented as zero order. As dose was increased, the absorption time also increased, and analgesics therapeutic efficacy exhibits longer duration. The distribution of sebacoyl dinalbuphine ester in animal was best fitted with a two-compartment model. As shown in Table 6, the half life for excretion was about 30 hours. It was much longer than that of Nalbuphine hydrochloride, which was about 1 hour. This is the reason why the preparation of polynalbuphine derivative has longer analgesic effect than traditional preparations of Nalbuphine hydrochloride.

Besides, the bioavailability of sebacoyl dinalbuphine ester was about 63 %. Data in Table 7 were compared with those of Table 6. Results showed similar AUC/Dose and similar total clearance (Clt) for two different dose administrations. Thus, sebacoyl dinalbuphine ester presented a linear kinetic, and the dose of sebacoyl dinalbuphine ester increased, the zero order absorption rate constant (Ko) also increased proportionally, and duration of zero order absorption(absorption time) was also proportionally increased.

The polynalbuphine derivative can be formulated into pharmaceutically acceptable long acting parenteral dosage forms or various pharmaceutical dosage forms for orally, oily parenteral administration and other administrations, which may be administered once a day, or once for several days. Even when large amounts are administered, the occurrence of untoward effects were minimized. The advantages of the present invention are described as above, such as long duration, untoward effects, and safety that should improve therapeutic quality. The dosing interval can be set longer than 24 hours instead of 3-5 hours for post-operation patient. For last cancer stage patients, administration of the present invention in dosage forms, instead of hospitalization, can yield the same therapeutic efficacy.

The polynalbuphine can be designed as a soft drug, which would release bioactive Nalbuphine in the animal body. In general, drugs are metabolized into active forms and inactivity forms. The polynalbuphine derivative soft drug would only release bioactive Nalbuphine in the animal body. New listed salt types, stereoisomers types, and prodrugs, are organized by US FDA and other countries. Since new drugs have to pass pharmaconogical and toxicological tests, usually only one of 7,000 new drugs in the R & D process makes a marketable drug. Since the polynalbuphine derivative soft drug releases solely bioactive Nalbuphine, it will make a marketable drug within a short period of time.

**EXAMPLES**

**Example 1 Preparation of propionyl Nalbuphine ester**

75 ml of methylene chloride and 3.57 g (0.01 mole) of Nalbuphine were added to a 250 ml round-bottomed flask. The flask was placed in an ice bath to keep it cool. The content was stirred, and 0.16 mole of triethylamine was gradually added. With rapid stirring, another 20 ml of methylene chloride solution containing 0.011 mole of propionic anhydride was added dropwise. Afterward, the mixture was stirred at room temperature for 1 hour. 20 ml of 10% sodium carbonate solution was added to neutralize the residual acid and to remove the water soluble impurities. Sodium sulfate was used to dehydrate the solution. After vaccum drying, a propionoyl MonoNalbuphine ester solid was obtained. The product was further purified by column chromatography.

**Examples 2 to 8 Preparation of Nalbuphine esters**

Procedures in example 1 were followed for preparing different MonoNalbuphine esters which are listed in Table 8.

**Example 9** **Preparation of adipoyl dinalbuphine ester**

5 g of Nalbuphine hydrochloride and 20 ml of dried dichloromethane were added into a round flask. The flask was placed in an ice bath. After 5 minutes, the content was stirred for 5 minutes, and 4.4 ml of triethylamine was gradually added. Another 5 ml of dichloromethane solution containing adipoyl chloride was added dropwise. The reaction mixture was stirred for 30 minutes. Then, the flask was removed from the ice bath, and was kept stirring for another 30 minutes under room temperature, followed by filtration to remove salts. The filtrate was added to 10 ml of dichloromethane, then washed with 10 ml of saturated saline, followed by adding 10 ml of 5% citric acid solution. Magnesium sulfate was used to dehydrate the solution and to concentrate the organic phase. Crystallization step in ethyl acetate and propanol for the organic phase was employed to obtain 4.2 g of adipoyl dinalbuphine ester. The melting point was determined to be 78 to 79 °C for the product.

**Example 10 to 14** **Preparation of other dinalbuphine esters**

Procedures in example 9 9 were followed for preparing different polynalbuphine esters which are listed in Table 9.

**Example 15** **Preparation of sebacoyl dinalbuphine ester (method I)**

20.2 g of sebacoyl acid and 200 ml of dimethylamine were added into a round flask. The flask was placed in an ice bath. After 5 minutes, the content was stirred for 30 minutes, and 32.4 g of 2-dipyridine carbonate was gradually added. 5 g of N, N-dimethylpyridine and 78.8 g of Nalbuphine hydrochloride were added, and the reaction mixture was further sitrred for 30 minutes. Then, the flask was removed from the ice bath to raise the temperature to room temperature, and was stirring for another 18 hours, followed by filtration to remove salts. When reaction was completed, acetic acid was added to neutralize and to control the pH value of the filtrate to about 7. Then, the solution was subjected to evaporation to remove dimethylamine. Upon removal, 500 ml of methylene chloride was added, followed by washing with 20 ml of saturated sodium chloride solution 3 times, and 20 ml of 5% citric acid 2 times. Magnesium sulfate was used to dehydrate the solution and to concentrate the organic phase. A yellowish solid of 50.2 g of sebacoyl dinalbuphine ester was obtained by crystallization in acetyl acetate and n-hexane for the organic phase.

**Example 16** **Preparation of sebacoyl dinalbuphine ester (method II)**

1.84 g octanoic acid and 5 ml of methylene chloride were added to a round flask. 3.93 g of Nalbuphine hydrochloride and triethylamine were put into flask. Stirring commenced for 30 minutes for reaction at room temperature. After the reaction, the flask was placed in an ice bath for 5 minutes. Upon the formation of salt, the mixture was filtered and concentrated. 5 ml of 10% sodium bicarbonate was added to dissolve oily stuff. Then, the pH of the solution was adjusted to 2.0 with 5 N HCl to allow sebacoyl dinalbuphine ester to precipitate. The precipitate was put into a round flask, and was dissolved with 5 ml of dimethylamine. The flask was placed in an ice bath. Then, 2.16 g of 2-dipyridine carbonate, 3.93 g of Nalbuphine hydrochloride, and 1 g of N,N dimethylpyridine were mixed in the ice bath, and stirring commenced for 30 minutes. The reaction was subsequently allowed to take place at room temperature for 18 hours. Then, the reaction solution was filtered under ice bath condition for 5 minutes for generating salts. The solution was dried, followed by adding 20 ml of dichloromethane, washing with 10 ml of saturated sodium chloride solution 3 times, and with 10 ml of 5% citric acid twice, Magnesium sulfate was used to dehydrate the solution and to concentrate the organic phase. A yellowish solid (sebacoyl dinalbuphine ester) was obtained by recrystallization in acetyl acetate and isopropanol for the organic phase.

**Example 17** **Preparation of 1,3-cyclohexane diacid dinalbuphine ester**

2 g of Nalbuphine hydrochloride and 10 ml of dichloremethane were added into a round flask. The flask was placed in an ice bath. After 5 minutes, the content was stirred, and 1.76 ml of triethylamine was gradually added. With rapid stirring, another 5 ml of dichloromethane solution containing 0.53 g of 1,3 cyclohexane diacid chloride was added dropwise. Upon reaction for 30 minutes, the flask was removed from the ice bath, and the reaction was continued for another 30 minutes. After the reaction was completed, the solution was placed in an ice bath for 5 minutes for generating salts. The solution was filtered to remove salts, and the filtrate was added with 20 ml of dichloromethane, followed by washing with 20 ml of saturated sodium chloride 3 times, and 20 ml of 5% citric acid twice. Magnesium sulfate was used to dehydrate the solution and to concentrate the organic layer. A white solid (1,3 cyclohexane diacid dinalbuphine ester) was obtained by column chromatography. The molecular formula was determined to be $C_{50}H_{62}N_2O_{10}$ for the product.

**Example 18** **Preparation of docosanodic dinalbuphine ester**

2 g of Nalbuphine hydrochloride and 10 ml of dichloremethane were added into a round flask. The flask was placed in an ice bath. After 5 minutes, 1.76 ml of triethylamine and 5 ml of dichloromethane solution containing 1.03 g of docosanodic diacid chloride were added dropwise. Upon reaction for 30 minutes, the flask was removed from the ice bath, and the reaction was kept going for another 30 minutes. After the reaction was completed, the solution was placed in an ice bath for 5 minutes for generating salts. The solution was filtered to remove salts, the filtrate was added with 20 ml of dichloromethane, followed by washing with 20 ml of saturated sodium chloride 3 times, and 20 ml of 5% citric acid twice. Magnesium sulfate was used to dehydrate the solution and to concentrate the organic layer. A white solid (docsanoidic dinalbuphine ester) was obtained by column chromatography. The molecular formula was determined to be $C_{64}H_{92}N_2O_{10}$, and the molecular weight was determined to be 1048.92 for the product.

**Example 19** **Preparation of 3,3-dimethylglutaric diacid dinalbuphine ester**

2 g of Nalbuphine hydrochloride and 10 ml of dichloremethane were added into a round flask. The flask was placed in an ice bath. After 5 minutes, 1.76 ml of triethylamine and 5 ml of dichloromethane solution containing 0.5 g of 3,3 dimethylglutaric acid chloride were added dropwise. Upon reaction for 30 minutes, the flask was removed from the ice bath, and the reaction was continued for another 30 minutes. After the reaction was completed, the solution was placed in an ice bath for 5 minutes for generating salts. The solution was filtered to remove salts, and the filtrate was added with 20 ml of dichloromethane, followed by washing with 20 ml of saturated sodium chloride 3 times, and 20 ml of 5% citric acid twice. Magnesium sulfate was used to dehydrate the solution and to concentrate the organic layer. A white solid (3,3-dimethylglutaric dinalbuphine ester) was obtained by column chromatography. The molecular formula was determined to be $C_{49}H_{62}N_2O_{10}$, and the molecular weight was determined to be 839.04 for the product.

**Example 20** **Preparation of trinalbuphine trimesoyl ester (method I)**

5 g of Nalbuphine hydrochloride and 20 ml of dichloromethane were added into a round flask. The flask was placed in an ice bath. After 5 minutes, 4.4 ml of triethylamine and 5 ml of dichloromethane solution containing 1.12 g of trimesoyl chloride were added separately. Upon reaction for 30 minutes, the flask was removed from the ice bath, and the solution was stirred for another 30 minutes. After the reaction was completed, the solution was placed in an ice bath for 5 minutes. The solution was filtered to remove salts, and the filtrate was added with 20 ml of dichloromethane, followed by washing with 20 ml of saturated sodium chloride 3 times, and 10 ml of 5% citric acid twice. Magnesium sulfate was used to dehydrate the solution and to concentrate the organic layer. A white solid (4.5 g of trinalbuphine trimesoyl ester) was obtained by column chromatography. The formula of the product was determined to be C50H56N2O10.

**Example 21** **Preparation of trinalbuphine trimesoyl ester ( method II)**

18.6 g of trimesoyl acid and 150 ml of dichloromethane were added to a round bottom flask. After 5 minutes, 20.3 g of 1-chloro-2,4-nitropyridine and 9.9 g of pyridine were added, and stirring was continued for 30 minutes. With rapid stirring, 118.05 g of Nalbuphine hydrochloride was added. Then, the flask was removed from the ice bath, and the reaction was continued for 18 hours. The solution was filtered to remove salts, the filtrate was added with 200 ml of dichloromethane, followed by washing with 20 ml of saturated sodium chloride 4 times, and 20 ml of 5% citric acid 3 times. Magnesium sulfate was used to dehydrate the solution and to concentrate the organic phase. A white solid of 9.1 g of trinalbuphine trimesoyl ester was obtained by recrystallization in n-hexane for the organic phase.

**Example 22** **Preparation of 1,3,5 cyclohexane triacid trinalbuphine ester**

2 g of dinalbuphine hydrochloride and 10 ml of dichloremethane were added into a round flask. The flask was placed in an ice bath. After 5 minutes, 1.76 ml of triethylamine and 5 ml of dichloromethane solution containing 0.46 g of 1,3,5 cyclohexane triacid chloride were added. Upon reaction for 30 minutes, the flask was removed from the ice bath, and stirring was continued for another 30 minutes. After the reaction was completed, the solution was placed in an ice bath for 5 minutes for generating salts. The solution was filtered, the filtrate was added with 20 ml of dichloromethane, followed by washing with 20 ml of saturated sodium chloride 3 times, and 20 ml of 5% citric acid twice. Magnesium sulfate was used to dehydrate the solution and to concentrate the organic layer. A white solid was obtained by column chromatography. The molecular formula was determined to be $C_{72}H_{87}N_3O_{15}$, and the molecular weight was determined to be 1234.49 for the product 3,3-dimethylglutaric dinalbuphine ester.

**Example 23** **Preparation of pyromellitoyl tetraNalbuphine ester**

2 g of dinalbuphine hydrochloride and 10 ml of dichloremethane were added into a round flask. After 5 minutes, 1.76 ml of triethylamine and 5 ml of dichloromethane solution containing 0.42 g of pyromellitoyl chloride were added dropwise. Upon reaction for 30 minutes, the flask was removed from the ice bath, and the reaction was continued for another 30 minutes. After the reaction was completed, the solution was placed in an ice bath for 5 minutes for generating salt. The solution was filtered, the filtrate was added with 20 ml of dichloromethane, followed by washing with 20 ml of saturated sodium chloride 3 times, and 20 ml of 5% citric acid twice. Magnesium sulfate was used to dehydrate the solution and to concentrate the organic phase. A white solid of 3,3-pyromellitoyl tetranalbuphine ester was obtained by column chromatography. The molecular formula was determined to be $C_{94}H_{106}N_4O_{20}$, and the molecular weight was determined to be 1611.88 for the product.

**Example 24** **Preparation of injection solution**

50 mg of sebacoyl dinalbuphine ester was added to 1 ml of sesame oil, and 1.8 mg of methyl paraben, 0.2 mg of propyl paraben, and 10 mg of pluronic F68 were subsequently added. The mixture was slightly shaked to effect a saturated injection solution.

**Example 25 to 29** **Preparation of injection solutions**

50 mg of sebacoyl dinalbuphine was added to 1 ml of sesame oil with different excipients listed in Table 4. Procedures in example 24 were followed to prepare saturated injections.

TABLE 1

**Adipoyl dinalbuphine ester**
FAB MS(M+1) : 825 (Int, 29.92% ),
1H-NMR(DMSO)
δ 6.80(d,1H), 6.62(d,1H), 4.8(s, 1H),
4.50(d,1H),
4.29(d,1H), 4.0(broad, 1H), 3.0~1.0(broad),
13C-NMR(DMSO)
δ 171, 149, 132, 131, 130, 122, 118, 91, 69, 85, 61.9, 59.9, 46, 42.9, 40.7, 40.3, 33.1, 32.8, 27.9, 26.5, 26.2, 23.7, 22.9, 18.3
IR- 3600-3100 cm-1 broad band,
3000-2800 cm-1 C-H stretch, 1745 cm-1 C-stretch.
3600-3100 cm-1 broad band,
3000-2800 cm-1 C-H stretch, 1745 cm-1 C-stretch.

**Suberoyl dinalbuphine ester**
MP:104-105 °C
FAB MS(M+1) 853 (Int, 100%)
1H-NMR (CDCl3
δ 6.76 (d, 1H), 6.62 (d, 1H), 5.05 (broad 1 H), 4.64 (d, 1H), 3.2-1.0 (broad),
13C-NMR(CDCl3)
δ 171.5(22), 148.4(3), 133.0(4), 131.3(12), 130.7(11), 121.5(1), 118.7(2), 91.5(5), 70.0(14), 66.5(6), 62.9 (9), 60.5(17), 46.1(13), 43.6(16), 33.7, 33.6 (23, 18), 32.0(15), 28.4(25), 26.7, 26.8(19,21), 26.2 (8), 24.6(24), 23.9 (7), 23.3 (10), 18.6 (20)
IR- 3700-3100 cm-1 broad band,
3000-2800 cm -1 CH stretch, 1760 cm-1 C-stretch

TABLE 1 (continued)

**Sebacoyl dinalbuphine ester**
MP:130-131 °C
FAB MS(M+1) 881 (Int 85%),
11H-NMR(DMSO)
δ 6.77(d, 1H) 6.60 (d,1H), 4.78 (s, 1H), 4.49 (d,1H), 4.24 (d,1H), 4.0(m), 3,1~1.0 (broad band),
13C-NMR(CDCl3)
δ 171.7 (22), 148.5(3), 133.0(4), 131.1(12), 130.8(11), 121.5(1), 118.8(2) 91.6(5), 70.0(14), 66.6(6), 62.9(9), 60.6(17), 46,1(13), 43.7(16), 33.9(23), 33.6(18), 32.0(15), Z8.8, 28.9(25, 26), 26.9, 26.7(19, 21), 26.2(8), 24.8 (24), 23.9(7), 23.3(10), 18.7(20),
IR- 3700~3100 cm-1(broad band) 3000-2800 CH stretch, 1760 C-stretch.

**Dodecanedioyl dinalbuphine ester**
MP:103-104 °C
FAB MS M+1 = 909 (Int 100%),
1H-NMR(DMSO)
δ 6.76 (d, 1H) , 6.62 (d,1H), 4.63 (s,1H), 4.17,
3.7~1.0 (broad band),
13C-NMR (CDCl3
δ 171.7(22), 148.5(3), 133.0(4), 131.4(12), 130.7(11), 121.5(1), 118.(2), 91.6(5), 70.0(14), 66.6(6), 63.0 (9), 60.6 (17), 46.1(13), 43.7(16), 33.9(23), 33.6(18), 32.0(15), 28.8, 28.9(25, 26), 26.9, 26.7 (19, 21), 26.3 (8), 24.9(24), 24.0(7), 23.4(10), 18.7(20),
IR- 3700-3100 cm-1 (broad band),
3000-2800 CH stretch, 1760 C-stretch.

**Isophthaloyl dinalbuphine ester**
MP:155~156 °C
FAB MS(M+1) 84.5 (Int 85%),
1H-NMR (DMSO)
δ 8.78(d, 1H), 8.46 (d, 1H), 7.86 (t, 1H), 7.01 (d, 1H), 6.69 (d,1H), 4.81 (s, 1H), 4.52 (d, 1H), 4.43 (d, 1H), 3.2-1.0 (broad band),
13C-NMR(DMSO)
δ 162.9(22), 149.4(3), 135~129 (23, 26, 4, 12, 11), 122.2(1), 118.3(2), 91.7(5), 69.3(14), IR-3700-3200 cm-1 (broad band),
3000~2750 C-H stretch, 1728 C-stretch.

**Phthaloyl dinalbuphine ester**
MP:137~138 °C
FAB MS(M+1) 845 (Int 31%),

1H-NMR (CDCl3)
δ 8.3 (s, 2H), 6.93 (d, 1H), 6.71(d, 1H), 5,1(s), 4.68 (d, 1H), 3.0~1.0 (broad band),
13C-NMR (CDCl3)
δ 163.3(22), 148.4(3), 133.3(4), 131.2(12), 130.4(11), 121.5(1), 118.9(2) , 91.8(5), 70.0(14), 66.5(6), 62.9(9), 60.5(17), 46,1(13), 43.7(16), 33.5(18), 32.0(15), 26.9, 26.7(19, 21), 26.2(8), 23.9(7), 23.3(10), 18.6(20),
IR- 3700~3200cm-1 (broad band),
3000-2750 CH stretch, 1728 C-stretch.

**1,3-cyclohexane diacid dinalbuphin ester**
FAB MS MH+ =850 (Int=100%).
1H-NMR(CDCl3)
δ 6.76(1H), 6.64(1H) , 4.84(1H), 4.17(1H), 3.15~1.0 (broad band)
13C-NMR(CDCl3)
δ 171.6(22), 148.4(3), 133.1(4), 131.2(12), 131.0(11), 121.4(1), 118.8(2), 91.7(5), 70.0(14), 66.5(6), 63.0(9), 60.6(17), 46.1(13), 43.7(16), 41.7(23), 33.6(18), 32.0 (15), 30.2(24), 26.9, 26.7, 26.3(19, 21, 25), 25.3(8), 23.9, 23.4(7,10), 18.7(20).
IR-(KBr) 3700~3100 cm-1 (broadband),1750 C=O Stretch.

**Docosaaodic dinalbuphine ester**
FAB MS MH+ =1234 (Int=30%)
FAB high resolution MS=C64H92N2O10

TABLE 1 (continued)

**3,3-Dimethylg1utaric diacid dinalbuphine ester**

FAB MS MH+1 =1234

1H-NMR(CDCl3)

δ 6.79 (d, 1H), 6.64(d, 1H), 4.64(d, 1H), 3,2-1.0 (broad band)

13C-NMR(CDCl3)

δ 171.6(22), 148.4(3), 133.1(4), 131.2(12) 131.0(11), 121.4(1), 118.8(2), 91.7(5), 70.0(14), 66.5(6), 63.0(9), 60.6 (17), 46.1(13), 43.7(16), 41.7(23), 33.6(18), 32.0 (15), 30.2(24), 26.9, 26.7, 26.3(19, 21, 25), 25.3(8), 23.9, 23.4 (7, 10), 18.7(20)

IR-(KBr) 3700~3100 cm-1 (broad band), 3000 ~2800 C-H stretch,

1750 C=O stretch

**Trimesoyl trinalbuphine ester**

MP:232-233 °C

FAB MS(M+) 1228 (Int 50%),

1H-NMR(CDCl3)

δ 9.24(s, 1H), 6.93(d, 1H), 6.71(d, 1H), 4.66(d, 1H), 3.2~1.0 (broad band),

13C-NMR(CDCl3)

δ 162.4(22), 148.3(3), 136.7~130.5(23, 24, 4, 11, 12), 121.4(1), 119.0(2), 91.9(5), 70.0(14), 66.6(6), 62.9(9), 60.5(17), 46.2(13), 43.7(16), 33.6(18), 32.1 (15), 26.9, 26.7(19, 21), 26.3(8), 23.9(7), 23.4(10), 18.7(20),

IR-3700~3200 cm-1 (broadband, aromatic C-H sketch), 3000~2800 cm-1, C-H stretch, 1740 cm-1 C=O Stretch.

**1,3,5,-Cyclohexane triacid trinalbuphine ester**

FAB MS MH+ = 1234 ,

1H-NMR(CDCl3)

δ 6.79(d,1H), 6.66(d,1H), 4.65(d, 1H), 3,2-1.0 (broad band)

IR-(KBr) 3700~3100 cm-1 (broad band),

3000~2800 C-H stretch,1750 C=O stretch.

**Pyromellitoyl tetranalbuphine ester**

FAB MS MH+ =1234 (Int= 48%),

1H-NMR(CDCl3)

δ 6.79(d,1H), 6.65(d,1H), 4.65(d,1H), 3,2-1.0(broad band)

13C-NMR(CDCl3)

δ 163.0(22), 148.5(3), 134.1(24), 133.2(4), 131.4(12), 131.0 (11), 121.7(1), 118.8(2), 91.8(5), 70.0(14), 66.5(6), 64.4(23), 62.9(9), 60.6(17), 46.2(13), 43.6(16), 33.6(18), 32.2(15), 27.0, 26.8(19, 21), 25.3(8), 23.8, 23.5(7, 10), 18.7(20)

IR-(KBr) 3700~3100cm-1 (broad band), 3000 ~ 2800 C-H stretch, 1750 C=O stretch.

Table 2

| Samples of analysis | Elimination Half-life(min) | Correlation coefficient of regression line |
|---|---|---|
| rat whole blood | 2.2± 0.5 | -0.98± 0.03 |
| rat plasma | 2.8± - | -0.994± - |
| rat red blood cell | 2.3±0.6 | -0.95±0.05 |
| rabbit whole blood | 14.9±1.3 | -0.985 ±0.003 |
| rabbit plasma | 6.98±0.03 | -0,992± 0.002 |
| rabbit red blood cell | 26.2±4.8 | -0.953 ± 0.03 |
| n=3<br>Data = mean± S.E | | |

Table 3

| Samples of analysis | Elimination Half-life(min) | Correlation coefficient of regression line |
|---|---|---|
| dog whole blood | 30.5±0.8 | -0.99943± 0.0008 |
| dog plasma | 27.8± 0.5 | -0.995± 0.003 |
| dog red blood cell | 33.4±1.4 | -0.966± 0.007 |
| human whole blood | 8.8±0.4 | -0.952 ±0.003 |
| human plasma | 9.0±0.4 | -0.93± 0.02 |
| human red blood cell | 7.7±0.4 | -0.94 ± 0.03 |
| n=3 | | |

Table 4

| Ingredients | Examples 24 | 25 | 26 | 27 | 28 | 29 |
|---|---|---|---|---|---|---|
| Sebacoyl Dinalbuphin Ester | 35mg /0.7g | 30mg /3.0g | 25mg /2.5. g | 25mg /2 5g | 30mg /3g | 50mg /5.0g |
| Methyl paraben | | | | | | 1.8mg /5.0g |
| Propyl paraben | | | | | | 0.2mg /0.02g |
| Pluronic F68 | | | | | | |
| Pluronic HSI 5 | | | | | | |
| Span 85 | | | | 0.2mg /0.02 | 0.2mg /0.02 | 0.2mg /0.02g |
| BHA | | | | | | |
| BHT | | | | | | |
| Cremophor EL | | | | | | |
| Sesame oil | 1ml /20ml | 1ml /100m | 1ml /100ml | 1ml /100m | 1ml /100m | 1ml /100ml |
| Peanut oil | | | | | | |

※Sebacoyl Dinalbuphine Ester.lot No. A9611501

Table 5

| Test Rseults of Sebacoyl Dinalbuphine ester oil Solution for I.M. injection | | | | |
|---|---|---|---|---|
| Examples | Appeaeranc | Identification | Assay | Note |
| 24 | pale yellow oily solution | pass | 99.5% | 2/8 vials with crystalline ppt |
| 25 | pale yellow oily solution | pass | 105.2% | 2/8 vials with crystalline ppt |
| 26 | pale yellow oily solution | pass | 97.0% 97.3% | |

Table 5 (continued)

| Test Rseults of Sebacoyl Dinalbuphine ester oil Solution for I.M. injection | | | | |
|---|---|---|---|---|
| Examples | Appeaeranc | Identification | Assay | Note |
| 27 | pale yellow oily solution, with whilte granulate ppt | pass | 101.2% 101.9% | 45°Ctest with crystalline ppt |
| 28 | pale yellow oily solution with crystalline ppt | pass | 103.0% 10.3.6% | 45°C, 1M, 1/2vial crystalline ppt |
| 29 | pale yellow oily solution | pass | 96.8% 95.7% | RT 1M tubid solution |
| 30 | pale yellow oily solution, with crystalline ppt | pass | 107.0% 106.7% | |
| 31 | pale yellow oily solution with crystalline ppt | pass | 102.2% | Temp ↑ crystalline ppt ↑ |
| 32 | pale yellow oily soltion | pass | 101.4% 101.3% | |
| 33 | pale yellow oily solution | pass | 101.5% | |
| 34 | pale yellow oily soltion | pass | 106.1% | |
| 35 | pale yellow oily solution | pass | 109.6% | SPAN 85 0.02% |

Table 6

| Parameters | Dog 1 | Dog 2 | Dog 3 | Dog 4 | Dog 5 | Mean | SD |
|---|---|---|---|---|---|---|---|
| A(μg/ml) | 20.9 | 14.1 | 9.5 | 14.1 | 16.5 | 15.0 | 4.2 |
| B(μg/ml) | 0.46 | 0.22 | 0.32 | 0.36 | 0.21 | 0.32 | 0.10 |
| α(l/h) | 8.54 | 6.85 | 23.02 | 4.76 | 6.74 | 9.98 | 7.41 |
| β(l/h) | 0.063 | 0.023 | 0.024 | 0.022 | 0.019 | 0.030 | 0.019 |
| $k_o$(μg/kg) | 129.9 | 149.9 | 199.1 | 249.5 | 174.9 | 180.6 | 46.4 |
| $t_{1/2,\ \alpha}$.(h) | 0.08 | 0.10 | 0.03 | 0.15 | 0.10 | 0.09 | 0.04 |
| $t_{1/2,\ \beta}$.(h) | 10.94 | 30.61 | 28.64 | 30.99 | 37.26 | 27.69 | 9.91 |
| Abs. time(h) | 164.5 | 95.6 | 49.3 | 58.0 | 95.6 | 92.6 | 45.4 |
| Vc(l/kg) | 13.88 | 10.21 | 14.95 | 10.65 | 24.50 | 14.84 | 5.77 |
| Vss(l/kg) | 360.9 | 450.1 | 428.1 | 303.6 | 1325 | 573.5 | 424 |
| k21(l/h) | 0.25 | 0.13 | 0.78 | 0.14 | 0.10 | 0.28 | 0.29 |
| k10(l/h) | 2.20 | 1.21 | 0.71 | 0.75 | 1.23 | 1.22 | 0.60 |
| k12(l/h) | 6.16 | 5.53 | 21.55 | 3.89 | 5.43 | 8.51 | 7.34 |
| Cmax(ng/ml) | 59 | 112 | 197 | 255 | 123 | 149 | 77 |
| AUC(h-μg/ml) | 9.73 | 11.88 | 13.75 | 19.23 | 13.64 | 13.65 | 3.52 |
| F(%) | 87.9 | 59.0 | 40.4 | 59.6 | 68.8 | 63.1 | 17.3 |
| AUMC($h^2$μg/ml) | 934.4 | 915.0 | 886.2 | 1217.4 | 1063.0 | 983.2 | 156.1 |
| MRT(H) | 84.3 | 78.8 | 61.1 | 62.7 | 79.1 | 73.2 | 10.6 |
| CLt(ml/h/kg) | 2.195 | 1.207 | 0.714 | 0.753 | 1.225 | 1.219 | 0.597 |

Table 7

| Parameters | Dog 2 | Dog 2 |
|---|---|---|
| Dose(mg/kg) | 24.3 | 81.0 |
| A(μg/ml) | 14.1 | 59.0 |
| B(μg/ml) | 0.22 | 0.51 |
| α(l/h) | 6.85 | 10.73 |

Table 7 (continued)

| Parameters | Dog 2 | Dog 2 |
|---|---|---|
| β(l/h) | 0,023 | 0,017 |
| $k_o$(μg/kgfh) | 149.9 | 354.2 |
| $t_{1/2,\ \alpha}$·(h) | 0.10 | 0.07 |
| $t_{1/2,\ \beta}$·(h) | 30.6 | 41.9 |
| Abs. time(h) | 95.6 | 168.0 |
| Vc(l/kg) | 10.21 | 10.21 |
| Vss(l/kg) | 450.1 | 860.6 |
| k21(l/h) | 0.13 | 0.11 |
| k10(l/h) | 1.21 | 1.64 |
| k12(l/h) | 5.53 | 8.99 |
| Cmax(ng/ml) | 112 | 204 |
| AUC(h-μg/ml) | 11.88 | 36.23 |
| AUC/D(h-kg/ml) | 0.478 | 0.488 |
| F(%) | 59.0 | 73.4 |
| AUMC($h^2$μg/ml) | 915 | 5026 |
| MRT(H) | 78.8 | 127.2 |
| CLt(ml/b/kg) | 1,207 | 1,641 |

TABLE 8

| Example | starting material | product |
|---|---|---|
| 2 | pivaloyl chloride | Nalbuphine pivalate |
| 3 | benzoyl chloride | Nalbuphine benzoate |
| 4 | heptanoyl chloride | Nalbuphine heptanoate |
| 5 | decanoyl chloride | Nalbuphine decanoate |
| 6 | behenic anhydride | Nalbuphine behenate |
| 7 | erucic anhydride | Nalbuphine erucicate |
| 8 | arachidic anhydride | Nalbuphine arachidate |

TABLE 9

| Example | starting material | product |
|---|---|---|
| 10 | suberoyl chloride | suberoyl dinalbuphine ester |
| 11 | sebacoyl chloride | sebacoyl dinalbuphin ester |
| 12 | Dodecanedioyl chloride | Dodecanedioyl dinalbuphin ester |
| 13 | Isophtbaloyl chloride | Isophthaloyl dinalbuphin ester |
| 14 | Phthaloyl chloride | Phthaloyl dinalbuphin ester |

## Claims

**1.** A polynalbuphine derivative comprising a compound having the following formula (I):

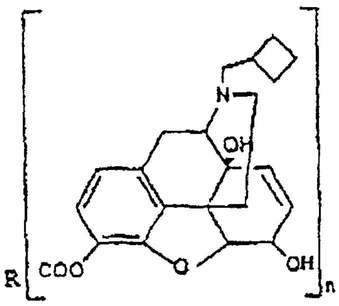

(I)

wherein n is an integer number ≧ 2; and R is a substituted or unsubstituted hydrocarbyl group having 1 to 40 carbon atoms wherein the substituents are selected from linear or branched alkyl or alkylene having 1 to 8 carbon atoms.

**2.** The polynalbuphine derivative as claimed in claim 1, wherein said hydrocarbyl group represents an aliphatic group consisting of linear or branched alkyl or alkylene having 1 to 40 carbon atoms, a substituted or unsubstituted alicyclic group having 3 to 12 carbons atoms, or a substituted or unsubstituted aromatic group having 6 to 40 carbon atoms, and n represents an integer in the range of 2 to 4.

**3.** The polynalbuphine derivative as claimed in claim 2, wherein said hydrocarbyl group represents a linear or branched alkyl or alkylene group having 20 to 35 carbon atoms, an alicyclic group having 3 to 8 carbon atoms and unsubstitutued or sustituted with a linear or branched alkyl or alkylene having 1 to 8 carbon atoms, or a phenyl unsubstitutued or substituted with an linear or branched alkyl or alkylene having 1 to 8 carbon atoms.

**4.** The polynalbuphine derivative as claimed in claim 1, wherein said compound of formula (I) is a product obtained by reacting Nalbuphine with a carbonyl group containing compound which is selected from a group consisting of a polybasic carboxylic acid, a polybasic carboxylic acid anhydride, and a polybasic carboxylic acid derivative containing at least two carbonyl chloride groups.

**5.** The polynalbuphine derivative as claimed in claim 4, wherein said polybasic carboxylic acid is selected from a group consisting of adipic acid, suberic acid, sebacic acid, dodecanedic acid, docosanic acid, 3,3-dimethyl glutaric acid, 1,3-cyclohexane dicarboxylic acid, isophthalic acid, phthalic acid, trimesic acid, 1,3,5-cyclohexane tricarboxylic acid, and pyromellitic acid.

**6.** The polynalbuphine derivative as claimed in claim 4, wherein said polybasic carboxylic acid anhydride is selected from a group consisting of adipic acid anhydride, suberic acid anhydride, sebacic acid anhydride, dodecanedic acid anhydride, docosanic acid anhydride, 3,3-dimethyl glutaric acid anhydride, 1,3-cyclohexane dicarboxylic acid anhydride, isophthalic acid anhydride, phthalic acid anhydride, trimesic acid anhydride, 1,3,5-cyclohexane tricarboxylic acid anhydride, and pyromellitic acid anhydride.

**7.** The polynalbuphine derivative as claimed in claim 4, wherein said polybasic carboxylic acid derivative is selected from a group consisting of adipoyl chloride, suberoyl chloride, sebacoyl chloride, dodecanedoyl chloride, docosanioyl chloride, 3,3-dimethyl glutaric diacid chloride, 1,3-cyclohexane dicarboxylic acid chloride, isophthaloyl chloride, phthaloyl chloride, trimesoyl chloride, 1,3,5-cyclohexane tricarboxylic acid chloride, and pyromellitoyl chloride.

**8.** A process for producing a compound having the following formula (I):

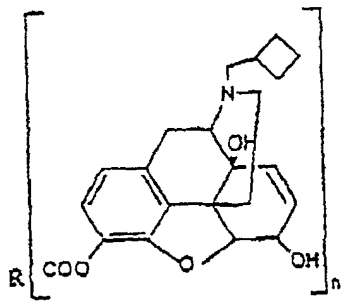

(I)

wherein n is an integer number ≧ 2; and R is a substituted or unsubstituted hydrocarbyl group having 1 to 40 carbon atoms, wherein the substituents are selected from linear or branched alkyl or alkylene having 1 to 8 carbon atoms, said process comprising the step of:

reacting Nalbuphine or Nalbuphine salt with a carbonyl group containing compound which is selected from a group consisting of a polybasic carboxylic acid, a polybasic carboxylic acid anhydride, and a polybasic carboxylic acid derivative containing at least two carbonyl chloride groups.

**9.** The process as claimed in Claim 8, wherein said polybasic carboxylic acid is selected from a group consisting of adipic acid, suberic acid, sebacic acid, dodecanedic acid, docosanic acid, 3,3-dimethyl glutaric acid, 1,3-cyclohexane dicarboxylic acid, isophthalic acid, phthalic acid, trimesic acid, 1,3,5-cyclohexane tricarboxylic acid, and pyromellitic acid.

**10.** The process as claimed in Claim 8, wherein said polybasic carboxylic acid anhydride is selected from a group consisting of adipic acid anhydride, suberic acid anhydride, sebacic acid anhydride, dodecanedic acid anhydride, docosanic acid anhydride, 3,3-dimethyl glutaric acid anhydride, 1,3-cyclohexane dicarboxylic acid anhydride, isophthalic acid anhydride, phthalic acid anhydride, trimesic acid anhydride, 1,3,5-cyclohexane tricarboxylic acid anhydride, and pyromellitic acid anhydride.

**11.** The process as claimed in Claim 8, wherein said polybasic carboxylic acid derivative is selected from a group consisting of adipoyl chloride , suberoyl chloride, sebacoyl chloride, dodecanedoyl chloride, docosanioyl chloride, 3,3-dimethyl glutaric diacid chloride, 1,3-cyclohexane dicarboxylic acid chloride, isophthaloyl chloride, phthaloyl chloride, trimesoyl chloride, 1,3,5-cyclohexane tricarboxylic acid chloride, and pyromellitoyl chloride.

**12.** The process as claimed in Claim 8, wherein the reaction of said Nalbuphine or said Nalbuphine salt with said carbonyl group containing compound is carried out in the presence of an organic solvent.

**13.** The process as claimed in Claim 12, wherein said organic solvent is selected from a group consisting of methylene chloride and ethylene chloride.

**14.** The process as claimed in Claim 13, wherein said organic solvent is methylene chloride.

**15.** The process as claimed in Claim 8, wherein said Nalbuphine salt is Nalbuphine hydrochloride.

**16.** The process as claimed in Claim 15, wherein the reaction of said Nalbuphine hydrochloride with said carbonyl group containing compound is carried out in the presence of a neutral agent for neutralizing said hydrochloride disassociated from said Nalbuphine hydrochloride in the reaction mixture.

**17.** The process as claimed in Claim 16, wherein said neutral agent is selected from a group consisting of a dimethylamine, trimethylamine, 2-pyridine carbonate, N,N-dimethylaminopyridine, N,N'-dicyclo-hexylcarbo-diimide, 4-dimethylaminopyridine, pyridine, and HOBT.

**18.** The process as claimed in Claim 17, wherein the reaction is carried out at a pH value of between 8 to 10.

**19.** The process as claimed in Claim 18, wherein the reaction is carried out at a temperature of between -10°C to 100°C.

**20.** The process as claimed in Claim 19, wherein said temperature is below room temperature and above -10°C.

**21.** The process as claimed in Claim 8, wherein the mole ratio of said nalbuphine to said carbonyl group containing compound is greater than 1:1 when n is equal to 2.

**22.** The process as claimed in Claim 8, wherein the mole ratio of said nalbuphine to said carbonyl group containing compound is greater than 2:1 when n is equal to 3.

**23.** The process as claimed in Claim 8, wherein the mole ratio of said nalbuphine to said carbonyl group containing compound is greater than 3:1 when n is equal to 4.

**24.** A pharmaceutical composition, comprising a compound having the following formula (I):

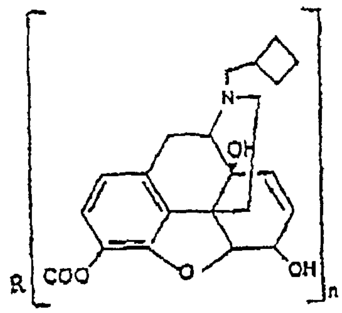

(I)

and a pharmaceutically acceptable carrier, wherein n is an integer number ≧ 2; and R is a substituted or unsubstituted hydrocarbyl group having 1 to 40 carbon atoms, wherein the substituents are selected from linear or branched alkyl or alkylene having 1 to 8 carbon atoms.

**25.** The composition as claimed in Claim 24, wherein said carrier is selected from a group consisting of sesame oil, soybean oil, and 5% peanut oil containing ethyl ester.

**26.** The composition as claimed in Claim 24, wherein said carrier is a diluent or an oil.

**27.** The composition as claimed in Claim 24, wherein said carrier is suspended or dissolved in oil.

**Patentansprüche**

**1.** Polynalbuphinderivat, das eine Verbindung der folgenden Formel (I)

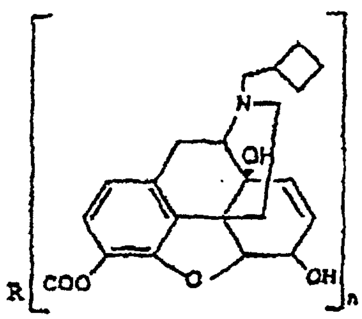

(I)

umfaßt, worin n eine ganze Zahl ≥ 2 ist und R eine substituierte oder unsubstituierte Hydrocarbylgruppe mit 1 bis 40 Kohlenstoffatomen ist, worin die Substituenten unter linearen oder verzeigtkettigen Alkyl- oder Alkylengruppen mit 1 bis 8 Kohlenstoffatomen ausgewählt sind.

**2.** Polynalbuphinderivat nach Anspruch 1, worin die Hydrocarbylgruppe eine aliphatische Gruppe, die aus linearem oder verzweigtkettigem Alkyl oder Alkylen mit 1 bis 40 Kohlenstoffatomen ausgewählt ist, eine substituierte oder unsubstituierte alizyklische Gruppe mit 3 bis 12 Kohlenstoffatomen oder eine substituierte oder unsubstituierte aromatische Gruppe mit 6 bis 40 Kohlenstoffatomen ist und n eine ganze Zahl im Bereich von 2 bis 4 bedeutet.

**3.** Polynalbuphinderivat nach Anspruch 2, worin die Hydrocarbylgruppe eine lineare oder verzweigtkettige Alkyl- oder Alkylengruppe mit 20 bis 35 Kohlenstoffatomen, eine alizyklische Gruppe mit 3 bis 8 Kohlenstoffatomen und unsubstituiert oder substituiert mit linearem oder verzweigtkettigem Alkyl oder Alkylen mit 1 bis 8 Kohlenstoffatomen oder ein Phenylrest unsubstituiert oder mit einem linearen oder verzweigtkettigen Alkyl oder Alkylen mit 1 bis 8 Kohlenstoffatomen ist.

**4.** Polynalbuphinderivat nach Anspruch 1, worin die Verbindung der Formel (I) ein Produkt ist, das man durch Umsetzung von Nalbuphin mit einer carbonylgruppenhaltigen Verbindung erhält, welche aus einer Gruppe ausgewählt ist, die aus einer mehrbasischen Carbonsäure, einem mehrbasischen Carbonsäureanhydrid und einem mehrbasischen Carbonsäurederivat, das wenigstens zwei Carbonylchloridgruppen enthält, ausgewählt ist.

**5.** Polynalbuphinderivat nach Anspruch 4, worin die mehrbasische Carbonsäure aus der Gruppe ausgewählt ist, die aus Adipinsäure, Suberinsäure, Sebazinsäure, Dodecandicarbonsäure, Docosansäure, 3,3-Dimethylglutarsäure, 1,3-Cyclohexandicarbonsäure, Isophthalsäure, Phthalsäure, Trimesinsäure, 1,3,5-Cyclohexantricarbonsäure und Pyromellitsäure besteht.

**6.** Polynalbuphinderivat nach Anspruch 4, worin das mehrbasische Carbonsäureanhydrid aus einer Gruppe ausgewählt ist, die aus Adipinsäureanhydrid, Suberinsäureanhydrid, Sebazinsäureanhydrid, Dodecandicarbonsäureanhydrid, Docosansäureanhydrid, 3,3-Dimethylglutarsäureanhydrid, 1,3-Cyclohexandicarbonsäureanhydrid, (sophthalsäureanhydrid, Phthalsäureanhydrid, Trimesinsäureanhydrid, 1,3,5-Cyclohexantricarbonsäureanhydrid und Pyromellitsäureanhydrid besteht.

**7.** Polynalbuphinderivat nach Anspruch 4, worin das mehrbasische Carbonsäurederivat aus einer Gruppe ausgewählt ist, die aus Adipoylchlorid, Suberoylchlorid, Sebazoylchlorid, Dodecanoylchlorid, Docosanoylchlorid, 3,3-Dimethylglutardicarbonsäurechlorid, 1,3-Cyclohexandicarbonsäurechlorid, Isophthaloylchlorid, Phthaloylchlorid, Trimesoylchlorid, 1,3,5-Cyclohexantricarbonsäurechlorid und Pyromellitoylchlorid besteht.

**8.** Verfahren zur Herstellung einer Verbindung der folgenden Formel (I)

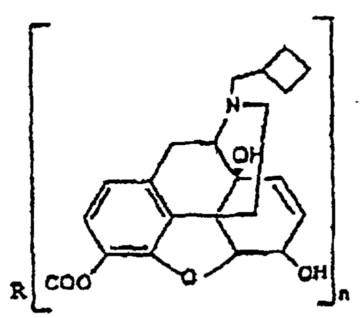

(I)

worin n eine ganze Zahl von ≥ 2 ist und R eine substituierte oder unsubstituierte Hydrocarbylgruppe mit 1 bis 40 Kohlenstoffatomen ist, worin die Substituenten unter linearen oder verzweigtkettigen Alkyl- oder Alkylengruppen mit 1 bis 8 Kohlenstoffatomen ausgewählt sind und das Verfahren folgende Stufen umfaßt, in denen man Naibuphin oder Naibuphinsalz mit einer Carbonylgruppen enthaltenden Verbindung reagieren läßt, die aus einer Gruppe ausgewählt wird, welche aus einer mehrbasischen Carbonsäure, einem mehrbasischen Carbonsäureanhydrid und einem mehrbasischen Carbonsäurederivat, das wenigstens zwei Carbonylchloridgruppen enthält, umsetzt.

**9.** Verfahren nach Anspruch 8, bei dem die mehrbasische Carbonsäure aus einer Gruppe ausgewählt wird, die aus Adipinsäure, Suberinsäure, Sebazinsäure, Dodecandicarbonsäure, Docosansäure, 3,3-Dimethylglutarsäure, 1,3-Cyclohexandicarbonsäure, Isophthalsäure, Phthalsäure, Trimesinsäure, 1,3,5-Cyclohexantricarbonsäure und Pyromellitsäure besteht.

**10.** Verfahren nach Anspruch 8, bei dem das mehrbasische Carbonsäureanhydrid aus der Gruppe ausgewählt wird, die aus Adipinsäureanhydrid, Suberinsäureanhydrid, Sebazinsäureanhydrid, Dodecandicarbonsäureanhydrid, Docosansäureanhydrid, 3,3-Dimethylglutarsäureanhydrid, 1,3-Cyclohexandicarbonsäureanhydrid, Isophthalsäureanhydrid, Phthalsäureanhydrid, Trimesinsäureanhydrid, 1,3,5-Cyclohexantricarbonsäureanhydrid und Pyromellitsäureanhydrid besteht.

**11.** Verfahren nach Anspruch 8, bei dem das mehrbasische Carbonsäurederivat aus der Gruppe ausgewählt ist, die aus Adipoylchlorid, Suberoylchlorid, Sebazoylchlorid, Dodecanoylchlorid, Docosanoylchlorid, 3,3-Dimethylglutardicarbonsäurechlorid, 1,3-Cyclohexandicarbonsäurechlorid, fsophthaloylchlorid, Phthaloylchlorid, Trimesoylchlorid, 1,3,5-Cyclohexantricarbonsäurechlorid und Pyromellitoyichlorid besteht

**12.** Verfahren nach Anspruch 8, bei dem die Umsetzung des Nalbuphins oder Nalbuphinsalzes mit der carbonylgruppenhaltigen Verbindung in Gegenwart eines organischen Lösungsmittels durchgeführt wird.

**13.** Verfahren nach Anspruch 12, bei dem das organische Lösungsmittel aus einer Gruppe ausgewählt wird, die aus Methylenchlorid und Ethylenchlorid besteht.

**14.** Verfahren nach Anspruch 13, bei dem das organische Lösungsmittel Methylenchlorid ist.

**15.** Verfahren nach Anspruch 8, bei dem das Naibüphinsalz Nalbuphinhydrochlorid ist.

**16.** Verfahren nach Anspruch 15, bei dem die Umsetzung des Nalbuphinhydrochlorids mit der carbonylgruppenhaltigen Verbindung in Gegenwart eines neutralen Mittels zum Neutralisieren des von dem Nalbuphinhydrochlarid in dem Reaktionsgemisch dissoziierten Hydrochlorid ausgeführt wird.

**17.** Verfahren nach Anspruch 16, bei dem das neutrale Mittel aus einer Gruppe ausgewählt wird, die aus einem Dimethylamin, Trimethylamin, 2-Pyridincarbonat, N, N.-Dimethylaminopyridin, N, N'-Dicyclohexylcarbodiimid, 4-Dimethylaminopyridin, Pyridin und HOBT besteht.

**18.** Verfahren nach Anspruch 17, bei dem die Umsetzung bei einem pH-Wert zwischen 8 und 10 durchgeführt wird.

**19.** Verfahren nach Anspruch 18, bei dem die Umsetzung bei einer Temperatur zwischen -10°C und 100°C ausgeführt wird.

**20.** Verfahren nach Anspruch 19, bei dem die Temperatur unter Raumtemperatur und oberhalb 10°C liegt.

**21.** Verfahren nach Anspruch 8, bei dem das Molverhältnis des Nalbuphins zu der carbonylgruppenhaltigen Verbindung größer als 1:1 ist, wenn n 2 bedeutet.

**22.** Verfahren nach Anspruch 8, bei dem das Molverhältnis des Nalbuphins zu der carbonylgruppenhaltigen Verbindung größer als 2:1 ist, wenn n 3 bedeutet.

**23.** Verfahren nach Anspruch 8, bei dem das Molverhältnis des Nalbuphins zu der carbonylgruppenhaltigen Verbindung größer als 3:1 ist, wenn n 4 bedeutet.

**24.** Pharmazeutische Zusammensetzung, die eine Verbindung der allgemeinen Formel (I)

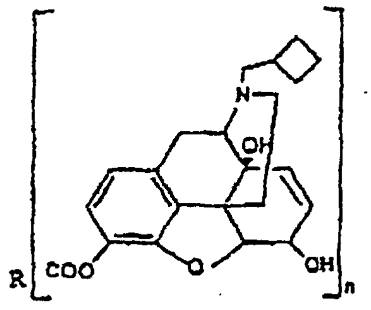

(I)

und einen pharmazeutisch verträglichen Träger umfaßt, worin n eine ganze Zahl ≥ 2 ist und R eine substituierte oder unsubstituierte Hydrocarbylgruppe mit 1 bis 40 Kohlenstoffatomen ist und worin die Substituenten unter linearen oder verzweigtkettigen Alkyl- oder Alkylengruppen mit 1 bis 8 Kohlenstoffatomen ausgewählt sind.

**25.** Zusammensetzung nach Anspruch 24, worin der Träger aus einer Gruppe ausgewählt ist, die aus Sesamöl, Sojabohnenöl und 5% Erdnußöl enthaltendem Ethylester besteht.

**26.** Zusammensetzung nach Anspruch 24, worin der Träger ein Verdünnungsmittel oder ein Öl ist.

**27.** Zusammensetzung nach Anspruch 24, bei der der Träger in Öl suspendiert oder aufgelöst ist.

## Revendications

**1.** Dérivé de polynalbuphine comprenant un composé répondant à la formule (I) suivante :

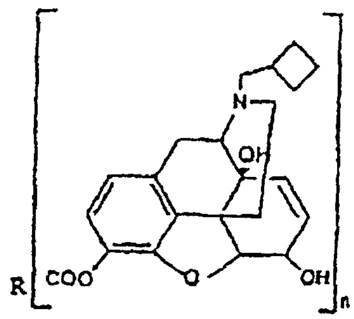

(I)

dans laquelle n représente un nombre entier égal ou supérieur à 2 ; et R représente un groupe hydrocarbyle, substitué ou non substitué, ayant 1 à 40 atomes de carbone, les substituants étant choisis entre des groupes alkyle ou alkylène linéaires ou ramifiés ayant 1 à 8 atomes de carbone.

**2.** Dérivé de polynalbuphine suivant la revendication 1, dans lequel ledit groupe hydrocarbyle représente un groupe aliphatique consistant en un groupe alkyle ou alkylène linéaire ou ramifié ayant 1 à 40 atomes de carbone, un groupe alicyclique, substitué ou non substitué, ayant 3 à 12 atomes de carbone, ou un groupe aromatique, substitué ou non substitué, ayant 6 à 40 atomes de carbone, et n représente un nombre entier de 2 à 4.

**3.** Dérivé de polynalbuphine suivant la revendication 2, dans lequel ledit groupe hydrocarbyle représente un groupe alkyle ou alkylène linéaire ou ramifié ayant 20 à 35 atomes de carbone, un groupe alicyclique ayant 3 à 8 atomes de carbone et qui est non substitué ou substitué avec un groupe alkyle ou alkylène linéaire ou ramifié ayant 1 à 8 atomes de carbone, ou un groupe phényle non substitué ou substitué avec un groupe alkyle ou alkylène linéaire ou ramifié ayant 1 à 8 atomes de carbone.

**4.** Dérivé de polynalbuphine suivant la revendication 1, dans lequel ledit composé de formule (I) est un produit obtenu en faisant réagir de la nalbuphine avec un composé contenant un groupe carbonyle qui est choisi dans le groupe consistant en un polyacide carboxylique, un anhydride de polyacide carboxylique et un dérivé de polyacide carboxylique contenant au moins deux groupes chlorure de carbonyle.

**5.** Dérivé de polynalbuphine suivant la revendication 4, dans lequel ledit polyacide carboxylique est choisi dans le groupe consistant en l'acide adipique, l'acide subérique, l'acide sébacique, l'acide dodécanoique, l'acide docosanoique, l'acide 3,3-diméthylglutarique, l'acide 1,3-cyclohexanedicarboxylique, l'acide isophtalique, l'acide phtalique, l'acide trimésique, l'acide 1,3,5-cyclohexane-tricarboxylique et l'acide pyromellitique.

**6.** Dérivé de polynalbuphine suivant la revendication 4, dans lequel l'anhydride de polyacide carboxylique est choisi dans le groupe consistant en l'anhydride d'acide adipique, l'anhydride d'acide subérique, l'anhydride d'acide sébacique, l'anhydride d'acide dodécanoïque, l'anhydride d'acide docosanoïque, l'anhydride d'acide 3,3-diméthylglutarique, l'anhydride d'acide 1,3-cyclohexane-dicarboxylique, l'anhydride d'acide isophtalique, l'anhydride d'acide phtalique, l'anhydride d'acide trimésique, l'anhydride d'acide 1,3,5-cyclohexane-tricarboxylique et l'anhydride d'acide pyromellitique.

**7.** Dérivé de polynalbuphine suivant la revendication 4, dans lequel ledit dérivé de polyacide carboxylique est choisi dans le groupe consistant en le chlorure d'adipoyle, le chlorure de subéroyle, le chlorure de sébacoyle, le chlorure de dodécanedioyle, le chlorure de docosanoyle, le chlorure de diacide 3,3-diméthylglutarique, le chlorure d'acide 1,3-cyclohexanedicarboxylique, le chlorure d'isophtaloyle, le chlorure de phtaloyle, le chlorure de trimésoyle, le chlorure d'acide 1,3,5-cyclohexane-tricarboxylique et le chlorure de pyromellitoyle.

**8.** Procédé pour la production d'un composé répondant à la formule (I) suivante :

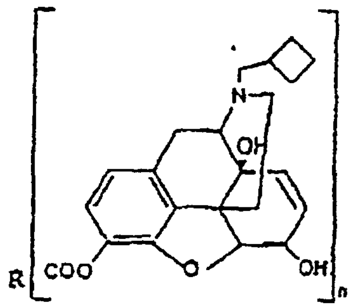

(I)

dans laquelle n représente un nombre entier égal ou supérieur à 2 ; et R représente un groupe hydrocarbyle, substitué ou non substitué, ayant 1 à 40 atomes de carbone, les substituants étant choisis entre des groupes alkyle ou alkylène linéaires ou ramifiés ayant 1 à 8 atomes de carbone, ledit procédé comprenant les étapes consistant :

à faire réagir de la nalbuphine ou un sel de nalbuphine avec un composé contenant un groupe carbonyle qui est choisi dans le groupe consistant en un polyacide carboxylique, un anhydride de polyacide carboxylique et un dérivé de polyacide carboxylique contenant au moins deux groupes chlorure de carbonyle.

**9.** Procédé suivant la revendication 8, dans lequel ledit polyacide carboxylique est choisi dans le groupe consistant en l'acide adipique, l'acide subérique, l'acide sébacique, l'acide dodécanedioïque, l'acide docosanoïque, l'acide 3,3-diméthylglutarique, l'acide 1,3-cyclohexane-dicarboxylique, l'acide isophtalique, l'acide phtalique, l'acide trimésique, l'acide 1,3,5-cyclohexanetricarboxylique et l'acide pyromellitique.

**10.** Procédé suivant la revendication 8, dans lequel ledit anhydride de polyacide carboxylique est choisi dans le groupe consistant en l'anhydride d'acide adipique, l'anhydride d'acide subérique, l'anhydride d'acide sébacique, l'anhydride d'acide dodécanedioïque, l'anhydride d'acide docosanoïque, l'anhydride d'acide 3,3-diméthylglutarique, l'anhydride d'acide 1,3-cyclohexane-dicarboxylique, l'anhydride d'acide isophtalique, l'anhydride d'acide phtalique, l'anhydride d'acide trimésique, l'anhydride d'acide 1,3,5-cyclohexane-tricarboxylique et l'anhydride d'acide pyromellitique.

11. Procédé suivant la revendication 8, dans lequel ledit dérivé de polyacide carboxylique est choisi dans le groupe consistant en le chlorure d'adipoyle, le chlorure de subéroyle, le chlorure de sébacoyle, le chlorure de dodécanedioyle, le chlorure de docosanoyle, le chlorure de diacide 3,3-diméthylglutarique, le chlorure d'acide 1,3-cyclohexanedicarboxylique, le chlorure d'isophtaloyle, le chlorure de phtaloyle, le chlorure de trimésoyle, le chlorure d'acide 1,3,5-cyclohexanetricarboxylique et le chlorure de piromellitoyle.

12. Procédé suivant la revendication 8, dans lequel la réaction de ladite nalbuphine ou dudit sel de la nalbuphine avec ledit composé contenant un groupe carbonyle est conduite en présence d'un solvant organique.

13. Procédé suivant la revendication 12, dans lequel ledit solvant organique est choisi dans le groupe consistant en le chlorure de méthylène et le chlorure d'éthylène.

14. Procédé suivant la revendication 13, dans lequel ledit solvant organique est le chlorure de méthylène.

15. Procédé suivant la revendication 8, dans lequel ledit sel de nalbuphine est le chlorhydrate de nalbuphine.

16. Procédé suivant la revendication 15, dans lequel la réaction dudit chlorhydrate de nalbuphine avec ledit composé contenant un groupe carbonyle est conduite en présence d'un agent neutre pour la neutralisation dudit chlorhydrate dissocié dudit chlorhydrate de nalbuphine dans le mélange réactionnel.

17. Procédé suivant la revendication 16, dans lequel ledit agent neutre est choisi dans le groupe consistant en la diméthylamine, la triméthylamine, le carbonate de 2-pyridine, la N,N-diméthylaminopyridine, le N,N'-dicyclohexylcarbodiimide, la 4-diméthylaminopyridine, la pyridine et le HOBT.

18. Procédé suivant la revendication 17, dans lequel la réaction est conduite à une valeur de pH comprise dans la plage de 8 à 10.

19. Procédé suivant la revendication 18, dans lequel la réaction est conduite à une température comprise dans l'intervalle de -10°C à 100°C.

20. Procédé suivant la revendication 19, dans lequel ladite température est inférieure à la température ambiante et supérieure à -10°C.

21. Procédé suivant la revendication 8, dans lequel le rapport molaire de ladite nalbuphine audit composé contenant un groupe carbonyle est supérieur à 1:1 lorsque n est égal à 2.

22. Procédé suivant la revendication 8, dans lequel le rapport molaire de ladite nalbuphine audit composé contenant un groupe carbonyle est supérieur à 2:1 lorsque n est égal à 3.

23. Procédé suivant la revendication 8, dans lequel le rapport molaire de ladite nalbuphine audit composé contenant un groupe carbonyle est supérieur à 3:1 lorsque n est égal à 4.

24. Composition pharmaceutique, comprenant un composé répondant à la formule (I) :

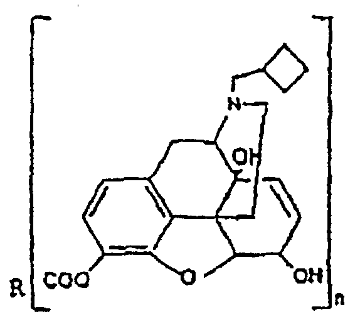

(I)

et un support pharmaceutiquement acceptable, formule dans laquelle n représente un nombre entier égal ou supérieur à 2 ; et R représente un groupe hydrocarbyle, substitué ou non substitué, ayant 1 à 40 atomes de carbone, les substituants étant choisis entre des groupes alkyle ou alkylène linéaires ou ramifiés ayant 1 à 8 atomes de carbone.

**25.** Composition suivant la revendication 24, dans laquelle ledit support est choisi dans le groupe consistant en l'huile de sésame, l'huile de soja et de l'huile d'arachide contenant 5 % d'un ester éthylique.

**26.** Composition suivant la revendication 24, dans laquelle ledit support est un diluant ou une huile.

**27.** Composition suivant la revendication 24, dans laquelle ledit support est mis en suspension ou dissous dans une huile.

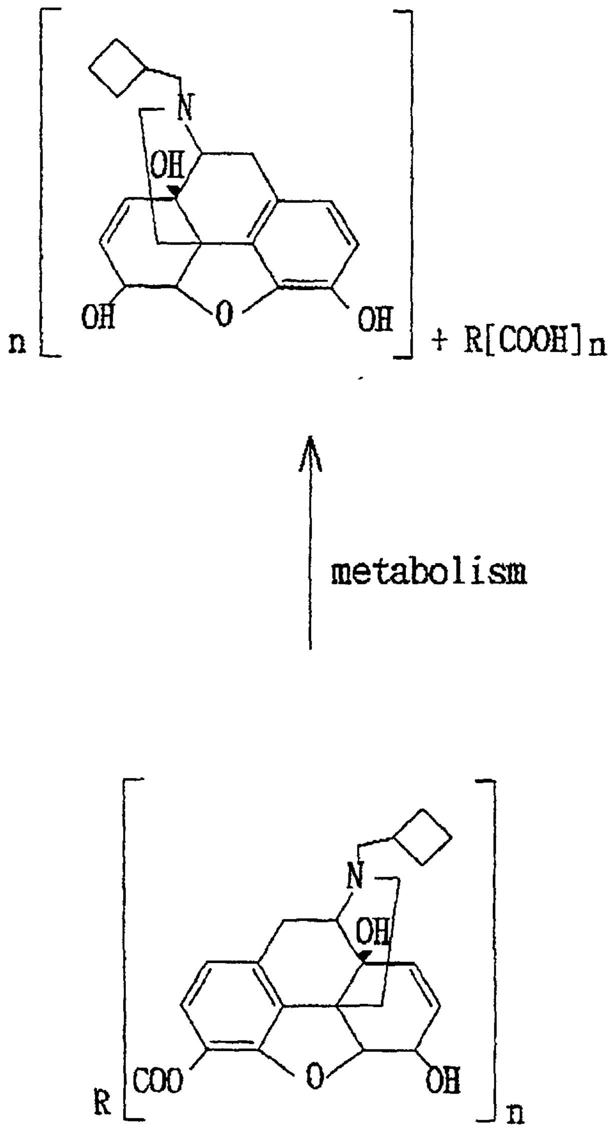


FIG. 1

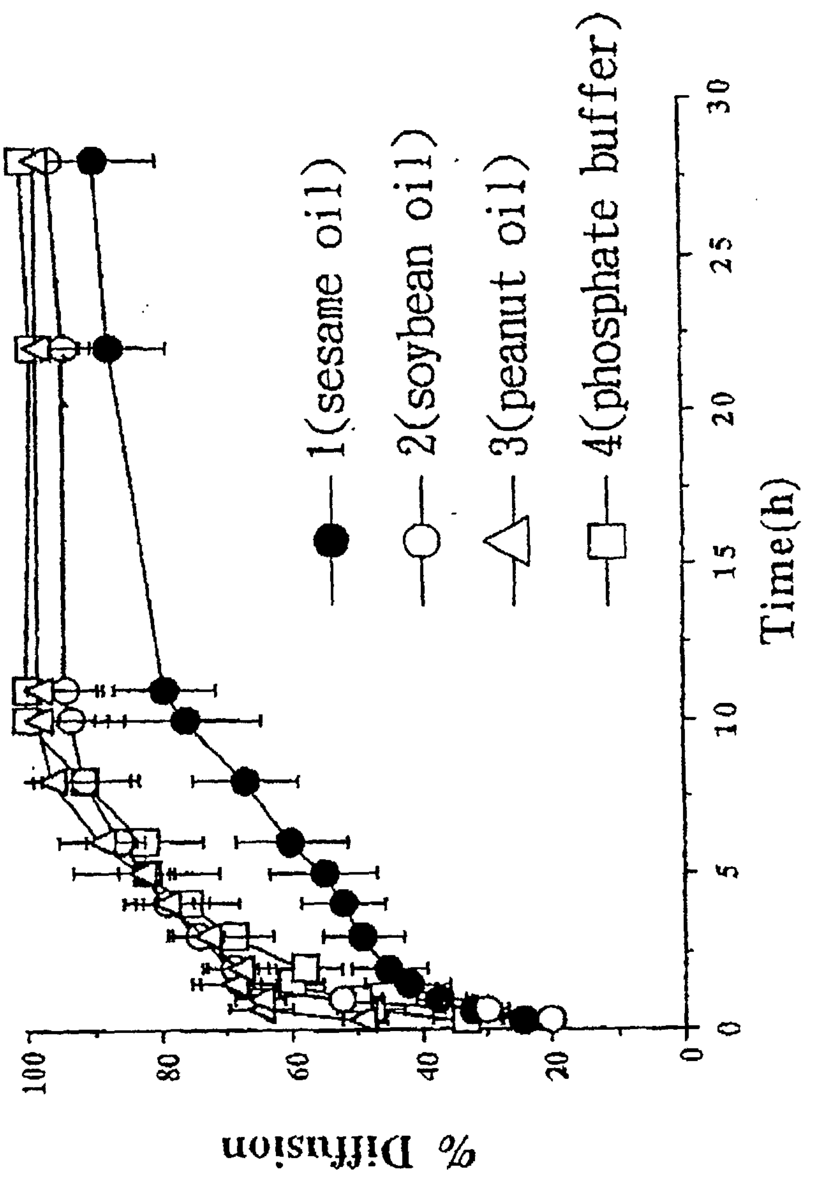
% Diffusion
100
80
60
40
20
0
0
5
10
15
20
25
30
Time(h)
1(sesame oil)
2(soybean oil)
3(peanut oil)
4(phosphate buffer)

FIG. 2

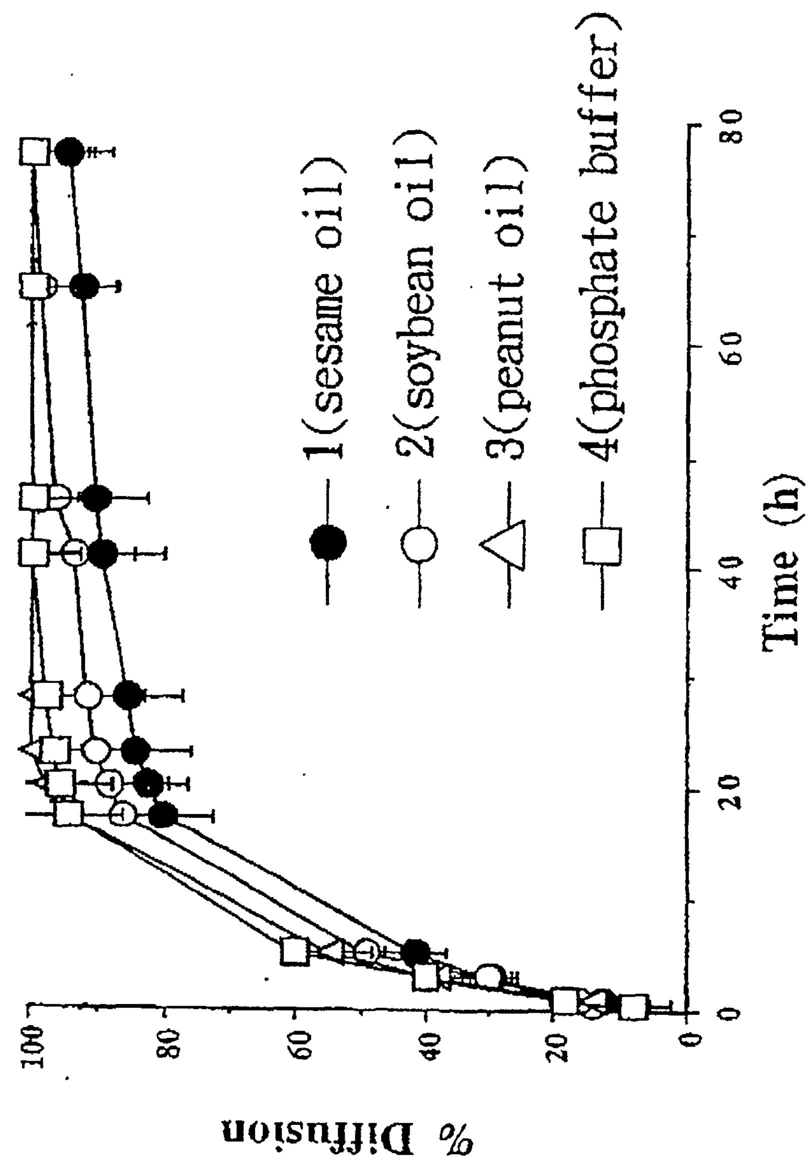
% Diffusion
100
80
60
40
20
0
0
20
40
60
80
Time (h)
1(sesame oil)
2(soybean oil)
3(peanut oil)
4(phosphate buffer)

FIG. 3

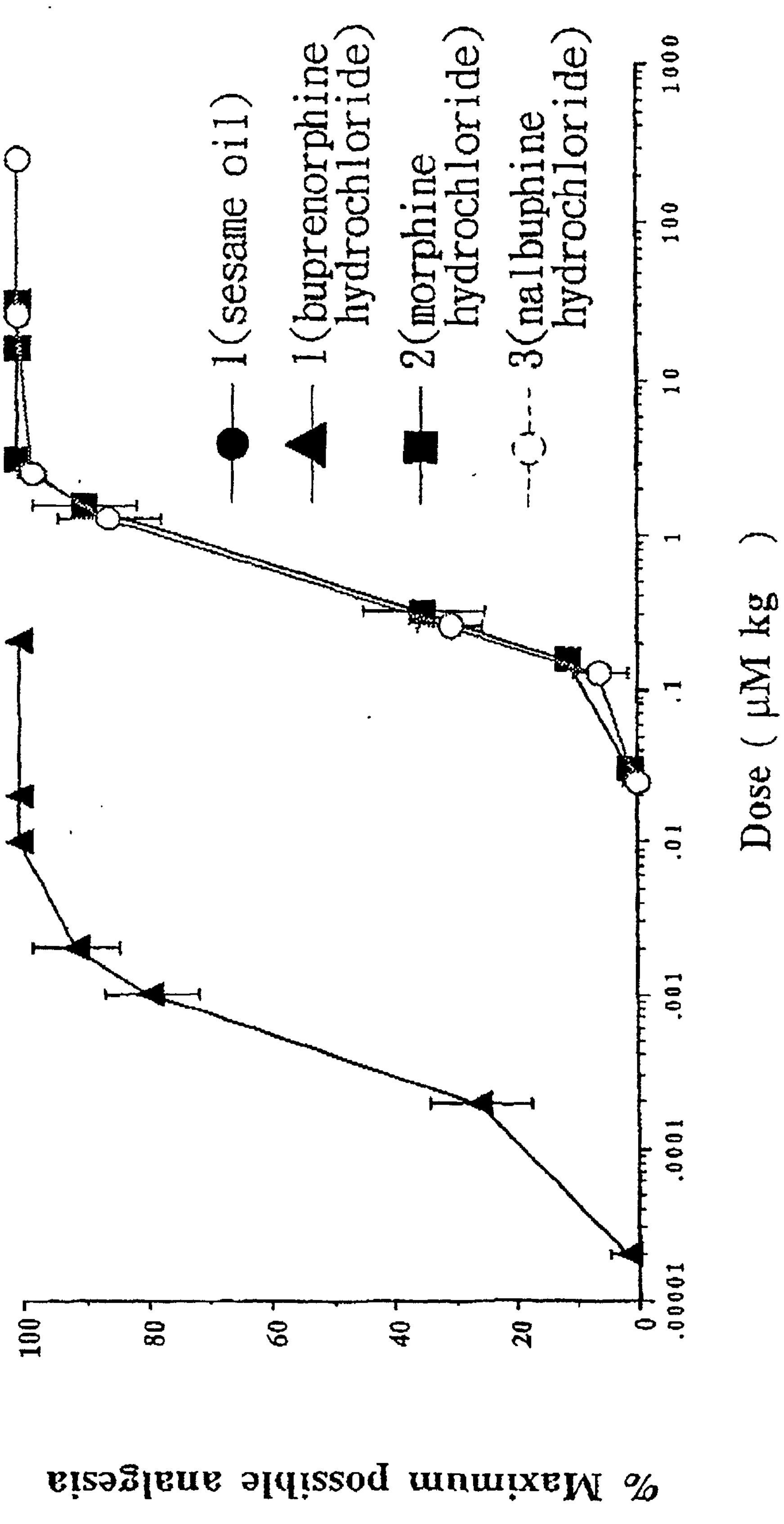

% Maximum possible analgesia
Dose ( μM kg )
100
80
60
40
20
0
.00001
.0001
.001
.01
.1
1
10
100
1000
1(sesame oil)
1(buprenorphine hydrochloride)
2(morphine hydrochloride)
3(nalbuphine hydrochloride)


FIG. 4

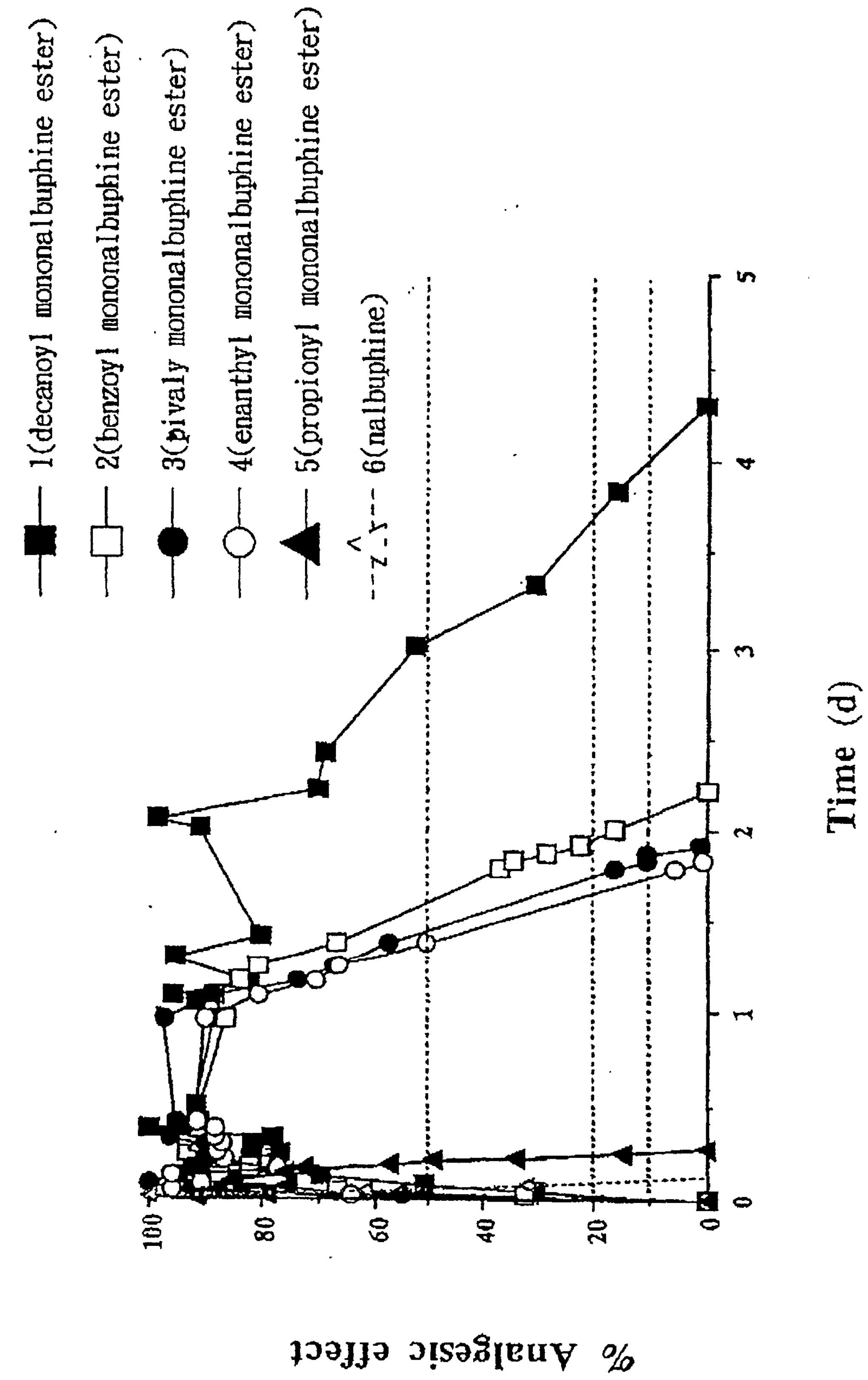
1(decanoyl mononalbuphine ester)
2(benzoyl mononalbuphine ester)
3(pivaly mononalbuphine ester)
4(enanthyl mononalbuphine ester)
5(propionyl mononalbuphine ester)
6(nalbuphine)
% Analgesic effect
100
80
60
40
20
0
0
1
2
3
4
5
Time (d)

FIG. 5

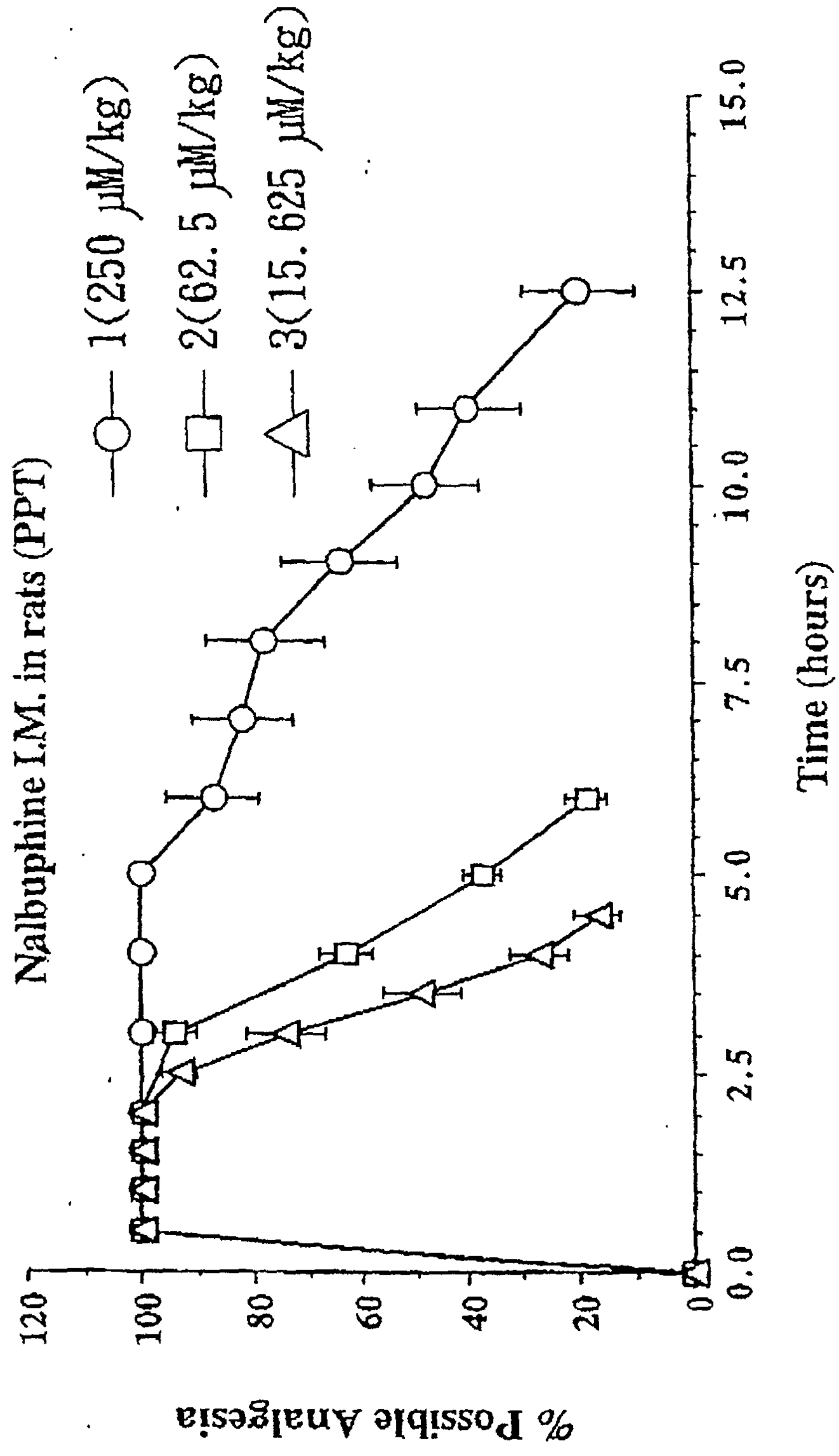
Nalbuphine I.M. in rats (PPT)
1(250 μM/kg)
2(62.5 μM/kg)
3(15.625 μM/kg)
% Possible Analgesia
120
100
80
60
40
20
0
0.0
2.5
5.0
7.5
10.0
12.5
15.0
Time (hours)

FIG. 6

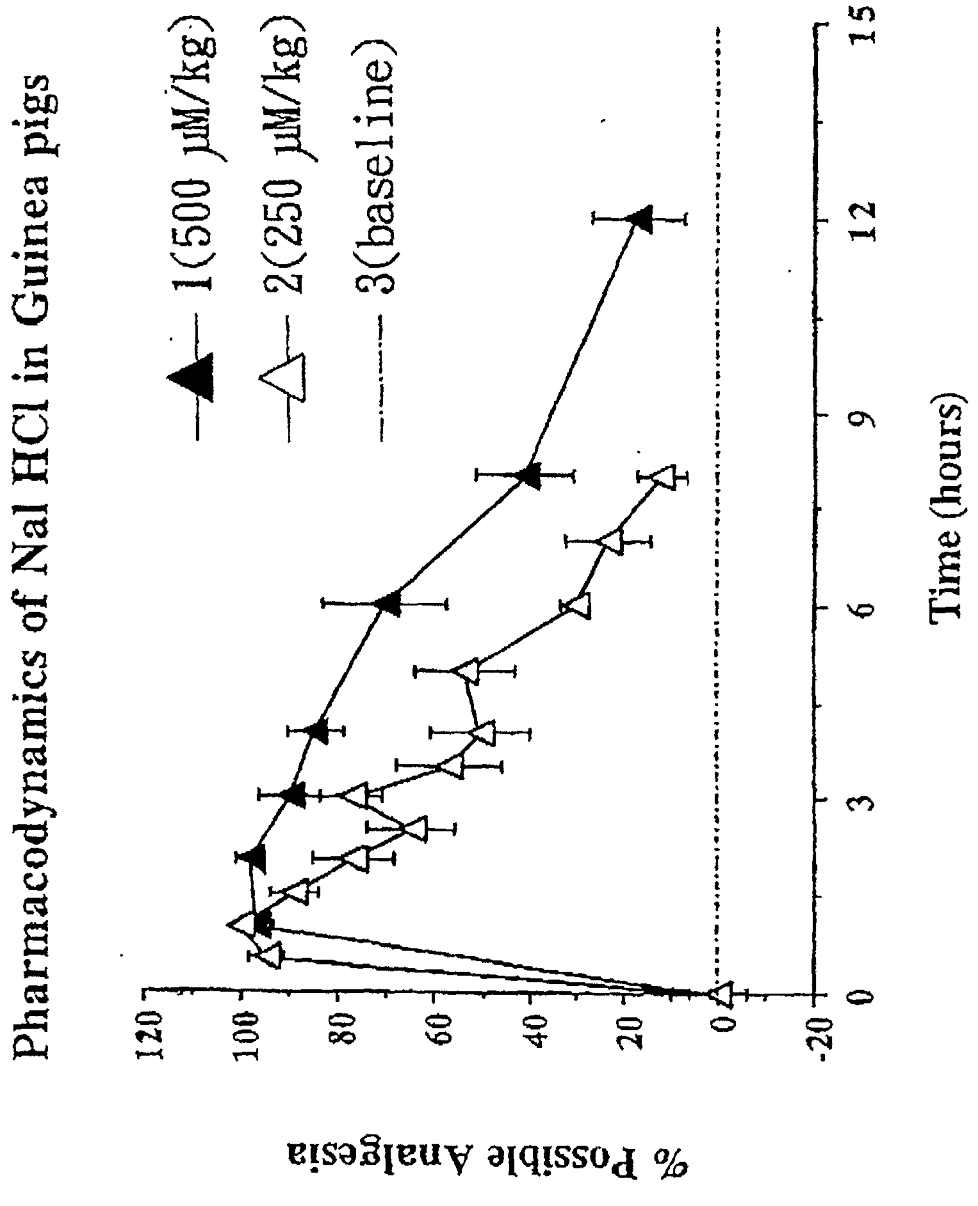
Pharmacodynamics of Nal HCl in Guinea pigs
1(500 μM/kg)
2(250 μM/kg)
3(baseline)
% Possible Analgesia
120
100
80
60
40
20
0
-20
0
3
6
9
12
15
Time (hours)

FIG. 7

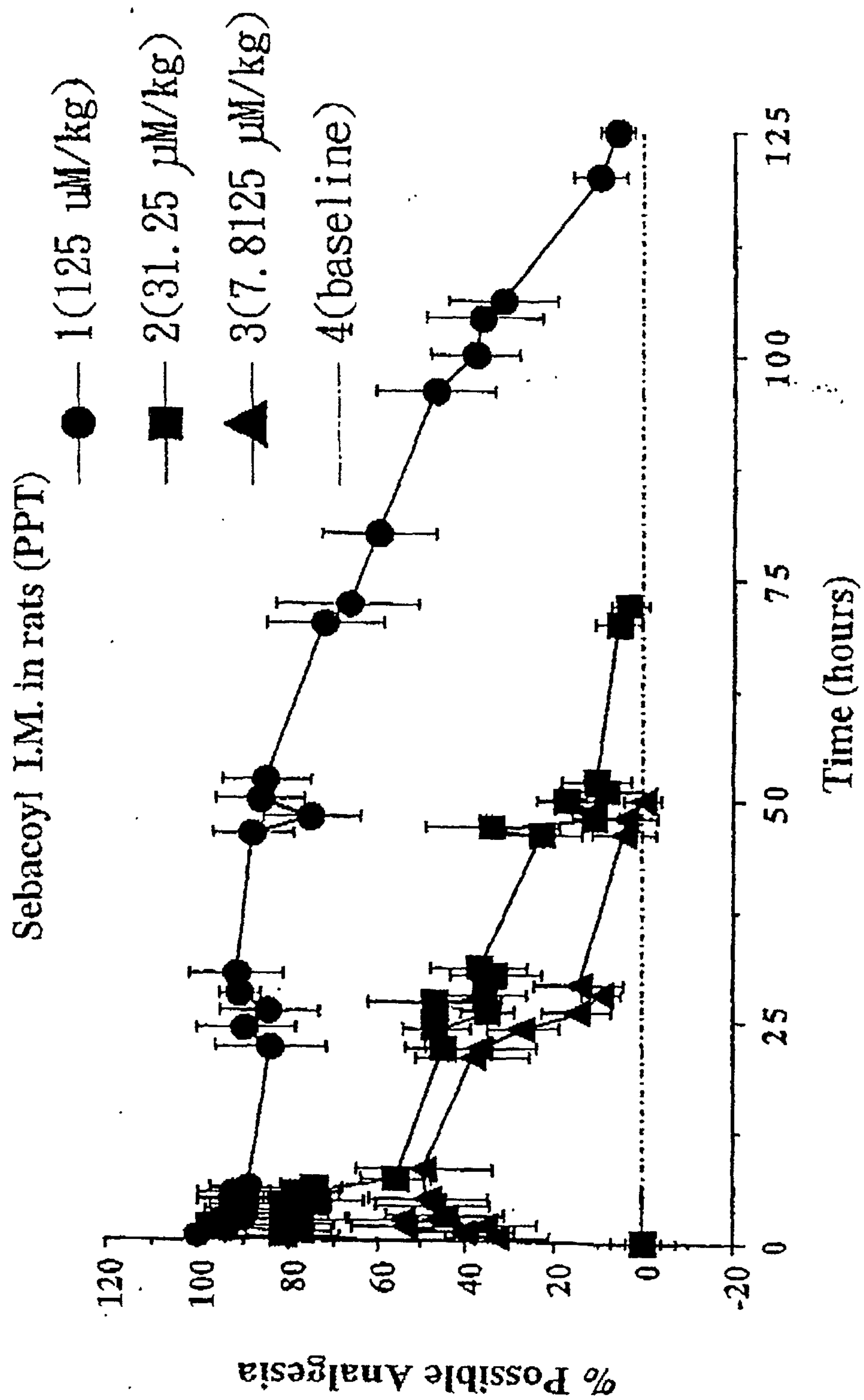
Sebacoyl I.M. in rats (PPT)
1(125 uM/kg)
2(31.25 μM/kg)
3(7.8125 μM/kg)
4(baseline)
% Possible Analgesia
120
100
80
60
40
20
0
-20
0
25
50
75
100
125
Time (hours)

FIG. 8

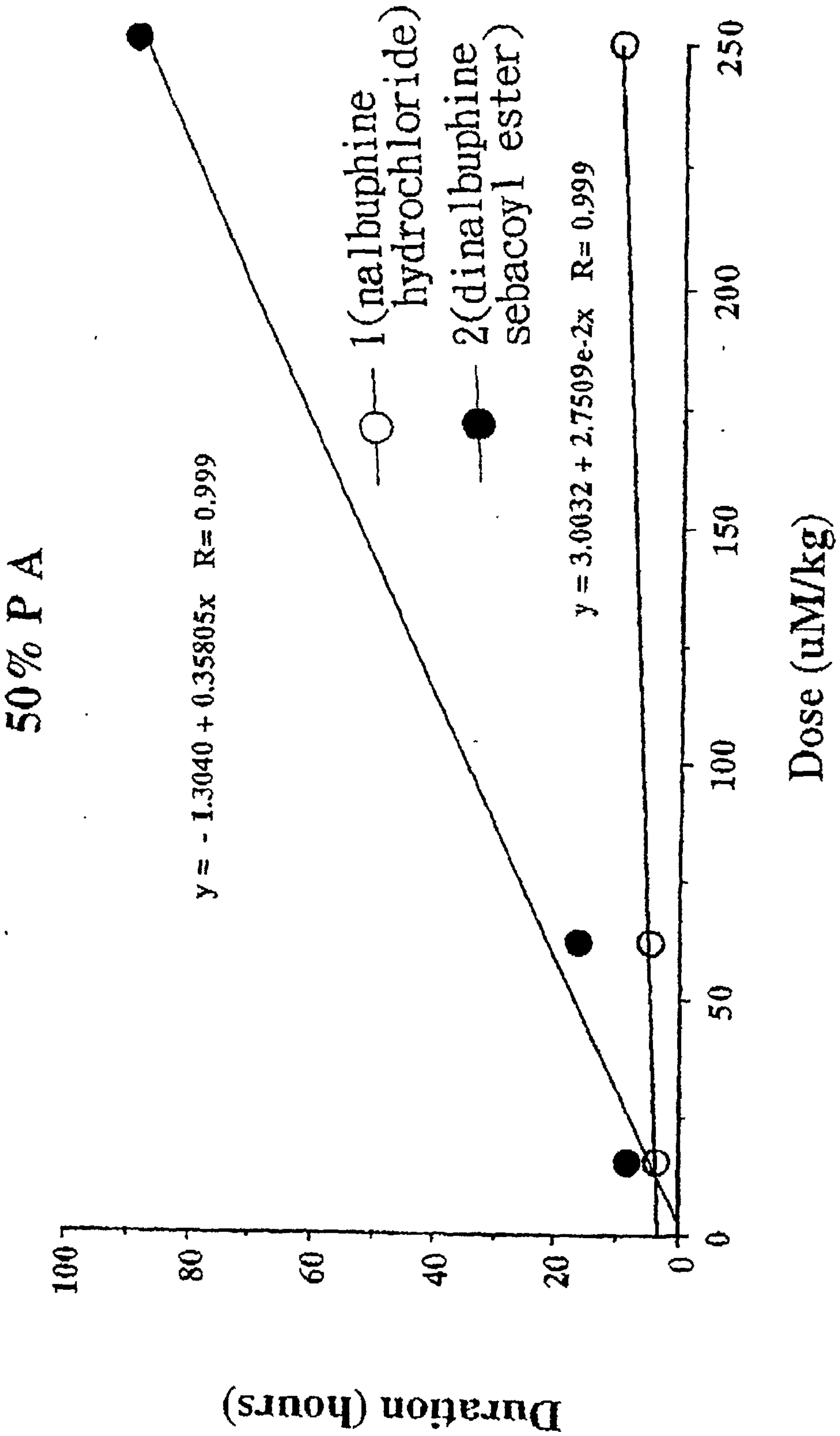
50% P A
y = - 1.3040 + 0.35805x R= 0.999
y = 3.0032 + 2.7509e-2x R= 0.999
1(nalbuphine hydrochloride)
2(dinalbuphine sebacoyl ester)
Duration (hours)
0
20
40
60
80
100
Dose (uM/kg)
0
50
100
150
200
250

FIG. 9

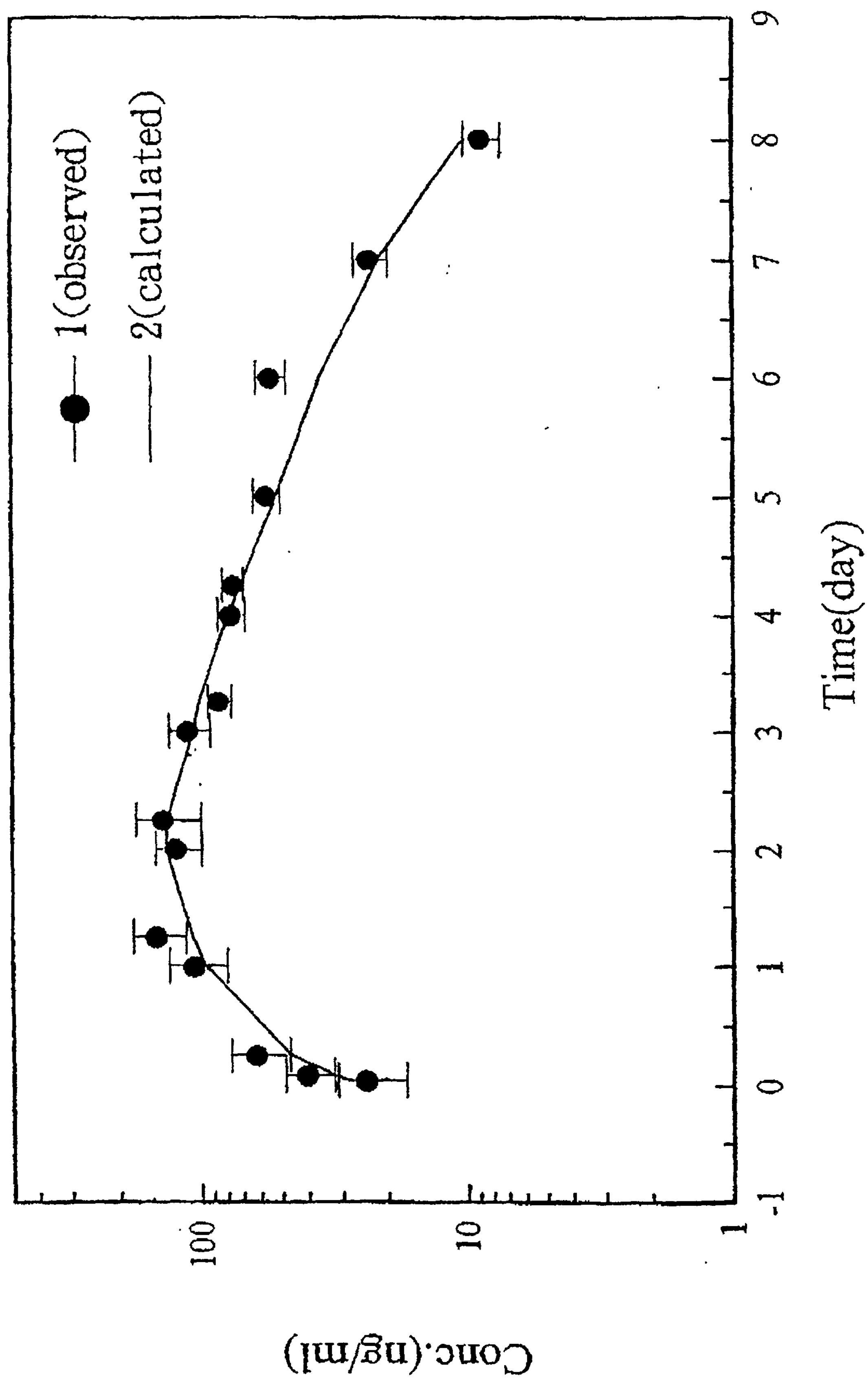

1(observed)
2(calculated)
Conc.(ng/ml)
100
10
1
Time(day)
-1
0
1
2
3
4
5
6
7
8
9


FIG. 10

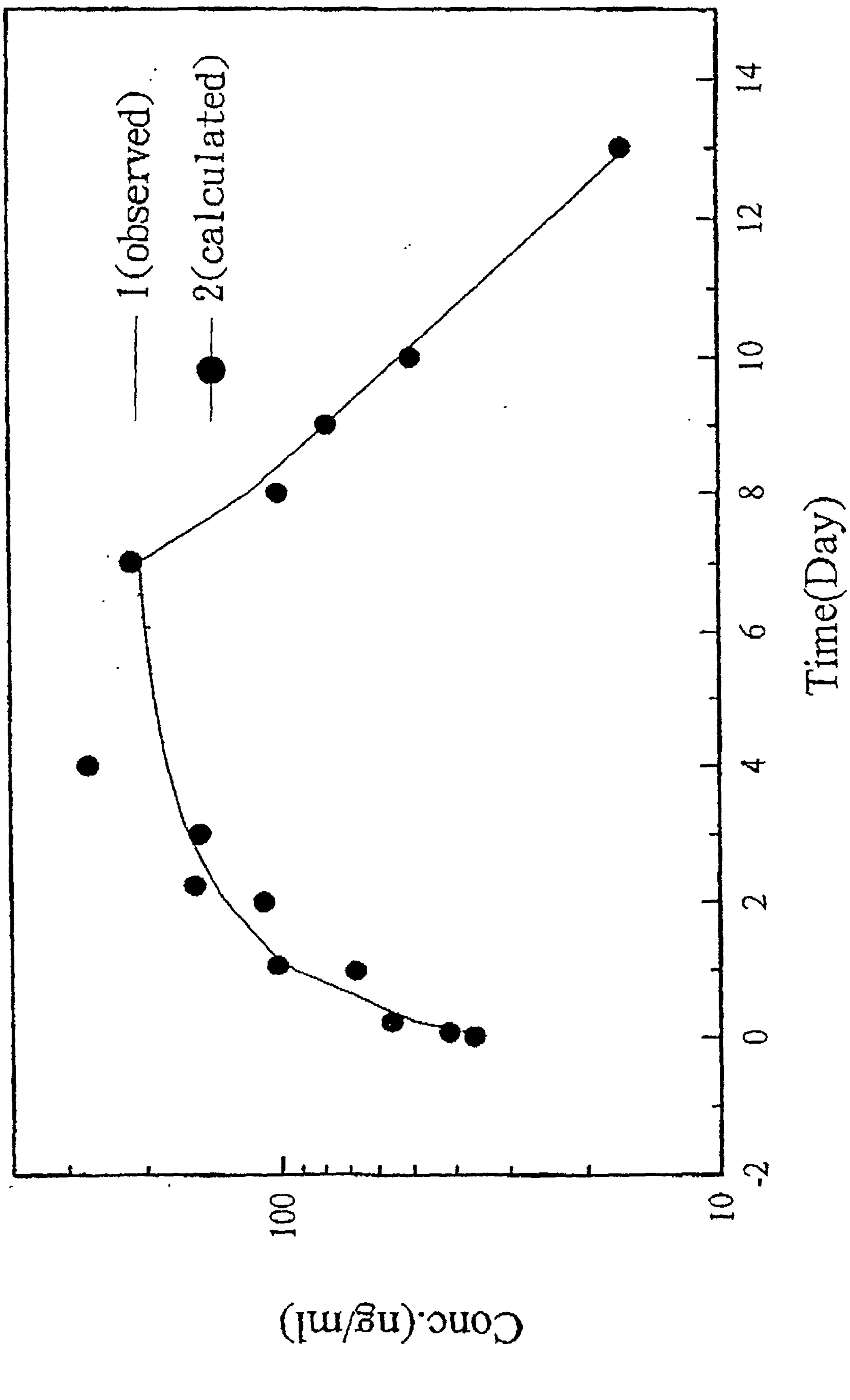

1(observed)
2(calculated)
Conc.(ng/ml)
10
100
Time(Day)
-2
0
2
4
6
8
10
12
14


FIG. 11